# EUROPEAN PATENT APPLICATION

(11) **EP 2 535 714 A1**
(43) Date of publication of application: **19.12.2012**
(21) Application number: 11170252.8
(22) Date of filing: 16.06.2011
(51) Int. Cl.: G01N 33/564, G01N 33/576

(54) **Biomarkers for autoimmune liver diseases and uses thereof**

(71) Applicant: Istituto Nazionale Di Genetica Molecolare-INGM, 20122 Milano (IT)
(72) Inventor: Abrignani, Sergio, 53040 Rapolano Terme (SI) (IT); Bombaci, Mauro, 20147 Milano (IT); Zingaretti, Chiara, 48121 Ravenna (IT)
(74) Representative: Capasso, Olga

(57) **Abstract**

The present invention relates to a method for diagnosis or prognosis of liver autoimmune diseases by means of detecting specific biomarkers in biological samples. The invention refers also to a method of monitoring an autoimmune liver disease pathology status after treatment with surgery and/or therapy in a subject with autoimmune liver disease, to kits and microarrays to perform said methods.

## Description

### FIELD OF INVENTION

The present invention relates to the field of immunodiagnostic and/or prognostic of liver autoimmune diseases.

### STATE OF THE ART

Autoimmune liver diseases include autoimmune hepatitis (AIH), primary biliary cirrhosis (PBC), and primary sclerosing cholangitis (PSC). Autoimmune hepatitis (AIH) has an incidence of 1-2 per 100,000 per year, and a prevalence of 1-10/100,000. As with most of the other autoimmune diseases, it affects women more often than men (80%). Histologically it is characterized by: interface hepatitis and plasma cell infiltration; hypergammaglobulinemia is often present; a number of autoantibodies can be detected such as antinuclear antibodies (ANA), anti-Smooth Muscle Antibody (SMA), liver/kidney microsomal antibody (LKM-1), LC1, anti-actin, anti-ASGPR (Bogdanos DP et al., Semin Liver Dis. 2009).

Primary Biliary Cirrhosis (PBC) is a non-suppurative destructive cholangitis leading to the T cell-mediated destruction of interlobular and septal bile ducts. It is characterized by: anti-mitochondrial antibodies -AMA- (∼90%) and intrahepatic cholestasis (increased alkaline phosphatase -Alk Ph-, normal ultrasonographic -US- scan). It affects women in 90% of cases. The prevalence is estimated at 0.6-40/100.000. Ursodeoxicholic acid has been shown to improve serum biochemistry, histology and patient's survival (Muratori L et al., Dig Liver Dis., 2010; Muratori L et al., Clin Liver Dis., 2008). Song Q et al. (J. Proteome Res, 2010) described the identification of highly specific biomarkers and their validation for AIH. This study demonstrates that the combination of six autoantigens can be used to diagnose AIH-positive serum samples and that these autoantigens can be effectively used in protein microarray assays, as well as, in traditional ELISA-based assays. US2009/0023162 discloses the methods for the identification of atypical antineutrophil cytoplasmic antibodies (ANCA), kits suitable for the same and application of said methods to the diagnosis of chronic inflammatory intestinal diseases and autoimmune liver diseases.

RU 2247387 (C1) provides a method involving determination of anti-mitochondrial antibodies, immunoglobulins such as IgA, IgM, IgG, gamma-globulins, anti-gliadin antibodies, and circulating immune complexes for the diagnosis of autoimmune liver injuries in patients with chronic hepatitis.

To date, however, there are no early and precise assays that can be used to identify individuals carrying or at risk of developing autoimmune liver disorders. An early diagnosis is clearly important.

### DESCRIPTION OF THE INVENTION

Though the identification of some autoantibodies is within prior art documents, there is still the urgent need to identify novel biomarkers, namely autoantibodies, to diagnose the liver autoimmune disease and/or to discriminate between autoimmune and other liver pathologies, and/or to monitor the efficacy of patient treatments of liver autoimmune disease and the disease progression.

In the present invention, a panel of 17 autoantigens was identified in patients with liver autoimmune diseases (autoimmune hepatitis (AIH) and Primary Biliary Cirrhosis (PBC)) by protein array. In addition, 4 out of the 17 autoantigens were also validated by Dissociation Enhanced Lanthanide FluorolmmunoAssay method (DELFIA^{®}), in patients diagnosed with liver autoimmune diseases, and showed individual sensitivities ranging from 50% to 65%. The combined assessment of the four autoantigens displays a 80% sensitivity and almost 100% specificity.

These four autoantigens represent novel markers of liver autoimmunity, such as AIH and PBC. These markers can display much higher sensitivity and specificity (Vs other diseases such as HCV and HBV) when compared to the benchmark markers (CYP2D6 & ASGPR, as described in the Methods section). Therefore, the autoantigens identified in the present invention are valuable tools for the diagnosis and/or prognosis of autoimmune liver diseases.

It is therefore an object of the present invention an *in vitro* method of diagnosis or prognosis of a liver autoimmune disorder in a subject, comprising the steps of:
a) contacting a biological sample from the subject with at least one protein selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof, under conditions appropriate for binding of autoantibodies present in the biological sample to said protein or immunological fragments or functional equivalents thereof, and
b) detecting the presence of bound antibodies wherein said detection is indicative of a liver autoimmune disorder or of a risk of developing a liver autoimmune disorder.

An immunological fragment refers to any synthetic or recombinant or proteolytic fragment of the proteins of the invention having the ability to recognize antibodies from a biological sample.

A functional equivalent is comprised in the group of synthetic or recombinant functional analogs invention having the ability to recognize antibodies directed against the protein from a biological sample.

In a preferred embodiment step a) is performed by contacting said biological sample with at least three proteins selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof.

In a further preferred embodiment step a) is performed by contacting said biological sample with at least four proteins selected from the group of 17 proteins having the amino acid sequences SEQ ID No 1, 2, 3, 5, 6, 7, 8, 9, 10, 11, 12, 14, 15, 16, 17, 22, 24 or immunological fragments or functional equivalents thereof.

In a further preferred embodiment said proteins or immunological fragments or functional equivalents thereof are comprised in the amino acid sequences of SEQ ID No. 1, 8, 17 and 22.

In a further preferred embodiment step a) is performed by contacting said biological sample with at least five proteins selected from the group of 17 proteins having the amino acid sequences SEQ ID No 1, 2, 3, 5, 6, 7, 8, 9, 10, 11, 12, 14, 15, 16, 17, 22, 24 or immunological fragments or functional equivalents thereof.

Even more preferably step a) is performed by contacting said biological sample with at least a further protein of the group of proteins or immunological fragments or functional equivalents thereof comprised in the amino acid sequences SEQ ID No. 2, 3, 5, 6, 7, 9, 10, 11, 12, 14, 15, 16, 24. Preferably the biological sample is comprised in the group of blood, serum, plasma, urine, saliva, mucus, or fractions thereof.

Preferably the liver autoimmune disorder is an Autoimmune Hepatitis (AIH) or a Primary Biliary Cirrhosis (PBC) disorder.

Preferably the biological sample is from an adult or from an adolescent. Preferably the detection of said bound autoantibodies is performed by means of binding to specific ligands. More preferably the ligands are conjugated with revealing means.

It is another object of the invention a method of monitoring an autoimmune liver disorder after treatment with surgery and/or therapy in a subject with said autoimmune liver disorder, comprising the step of following the modulation of antibodies as disclosed.

In a preferred aspect said proteins or immunological fragments or functional equivalents thereof are displayed on one or more protein microarrays.

It is another object of the invention a protein microarray comprising at least the proteins as defined or immunological fragments or functional equivalents thereof.

It is another object of the invention a solid support for an immunodiagnostic assay comprising at least the proteins as defined or immunological fragments or functional equivalents thereof.

It is another object of the invention a immunodiagnostic kit comprising the above solid support and detecting means.

In the present invention a subject with autoimmune liver disease is named "Al or hepatic autoimmunity patient" and is affected by any autoimmune liver disease, including AIH or PBC.

HCV patients are patients diagnosed with hepatitis C and displaying autoreactive antibodies, or patients with HCV infection or patients with hepatitis C and extrahepatic autoimmune diseases such as crioglobulinemia or thyroid dysfunctions.

The invention will be now described by means of non limiting examples referring to the following figures.

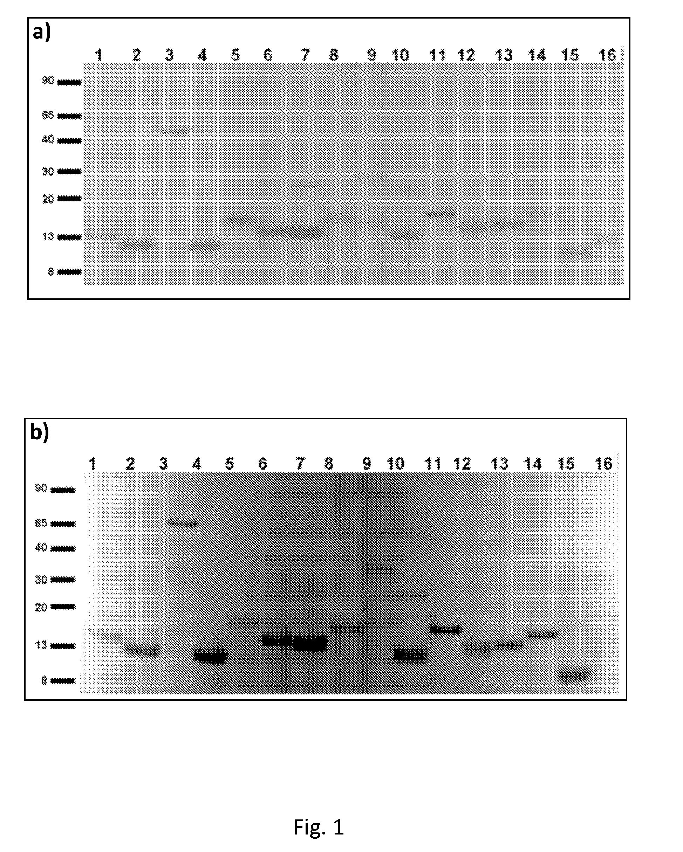

**Figure 1**: **a)** SDS-PAGE of a representative set of purified recombinant human proteins stained with Coomassie blue, or **b)** subjected to Western Blot analysis using an anti-histidine (His) tag monoclonal Antibody (mAb); molecular weight markers are shown on the left in kDa.

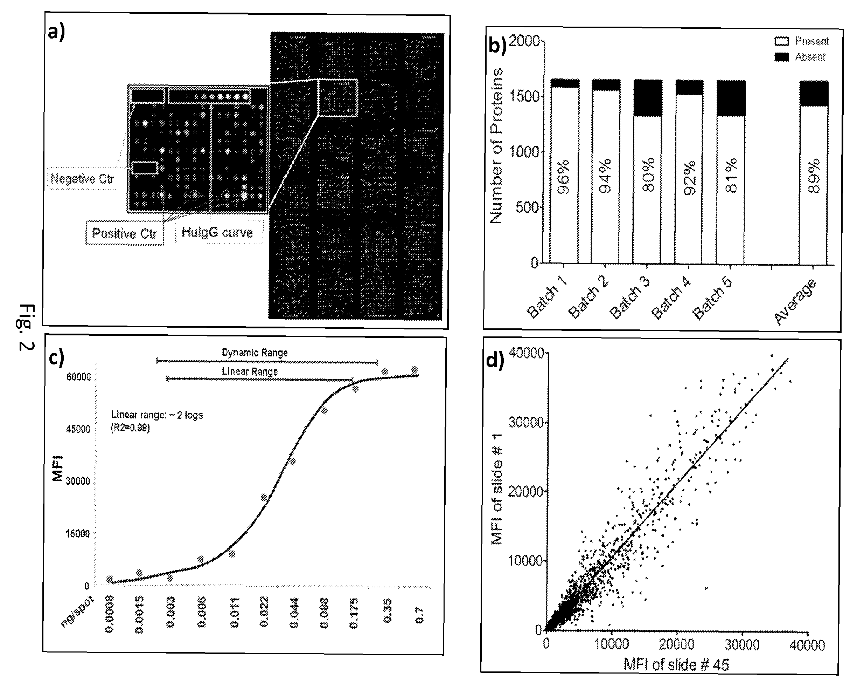

**Figure 2**: **a)** A human protein microarray containing 1658 His-tagged recombinant proteins was probed with an anti-His mAb followed by a secondary α-mouse antibody Alexa-647-labeled. The whole microarray included 24 grids (as the one shown in the enlarged box) each one containing proteins and control spots (positive Ctr and negative Ctr frames). Human IgG calibration curve (HulgG curve) used for data normalization is also shown; proteins and controls were always spotted in quadruplicates, while IgG were spotted in eight replicates. **b)** Number of proteins detected with the anti-His mAb. About 90% of proteins were spotted with success on the slides (i.e about 90 % of the proteins produced signals that were significantly above the background signal). Histograms: Proteins were considered "*Present*" when at least two out of the four replicates gave a signal above the background, otherwise they were considered "*Absent*". **c)** Plot of the control human IgG curve. The dots correspond to the experimental average signal (Mean Fluorescence Intensity, y-axis) detected for each IgG concentration reported on the x-axis in nanograms per spot (ng/spot), while the continuous line corresponds to the interpolated resulting sigmoid curve.
   *Dynamic Range:* refers to a range of values that can be measured by a signal (i.e. Mean Fluorescence Intensity).
   *Linear Range:* is the range of concentration over which the signal intensity is linear, i.e. directly proportional to the spot concentration. **d)** Correlation among spot intensities of two different slides (Slide 1 Vs Slide 45) of the same batch. The scatter plot indicates a positive correlation. The correlation coefficient is 0.9, indicating a high reproducibility of the signals derived from the proteins spotted.

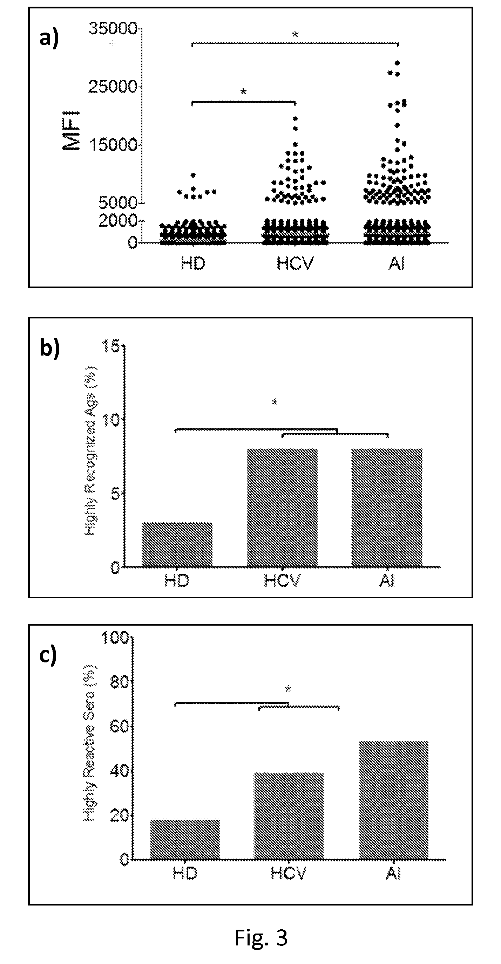

**Figure 3**: **a)** Comparison of Mean Fluorescence Intensities (MFIs) of total proteins spotted against three sera populations: Healthy Donor (HD), Hepatitis C Virus (HCV) and autoimmune liver disease (Al) patients. Each dot represents the MFI of a single protein. A cut-off value ≥ 4.000 was used to score a protein as positive. **b)** Percentage of antigens recognized by more than 15% of the sera tested; the threshold was determined on the basis of the average HD recognition. **c)** Percentage of reactive sera for each population (i.e. sera reacting with more than 3% of the proteins screened; the threshold was determined on the basis of the average HD reactivity).
   Asterisks: statistical significance, Student's t-test (pval <0.01) in A); Fisher's exact test (pval <0.01) in B) and C).

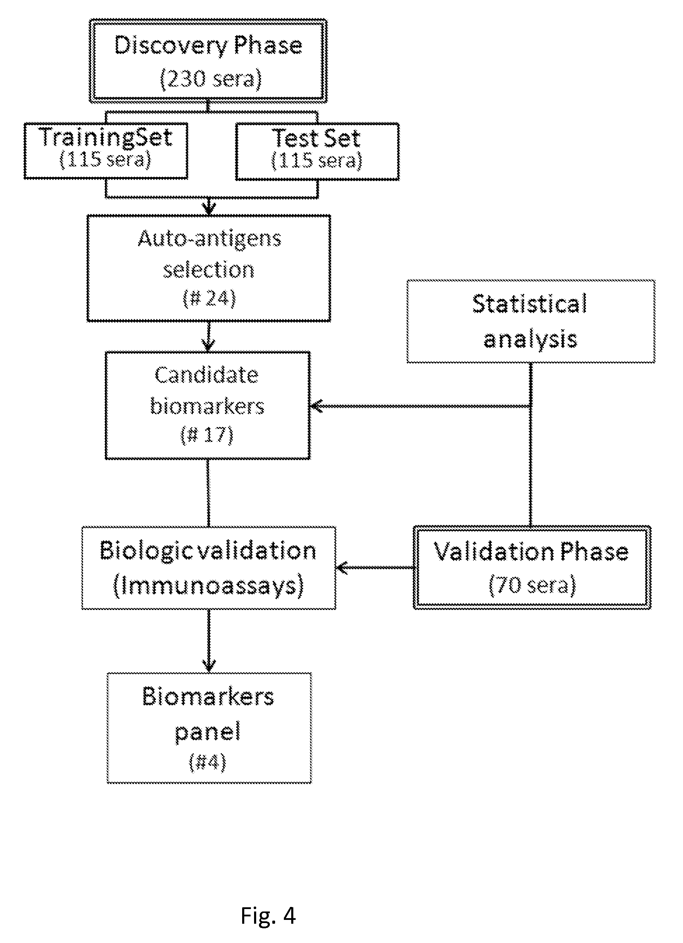

**Figure 4****:** Flowchart describing the selection of the autoantigens, as candidate biomarkers of the present invention.

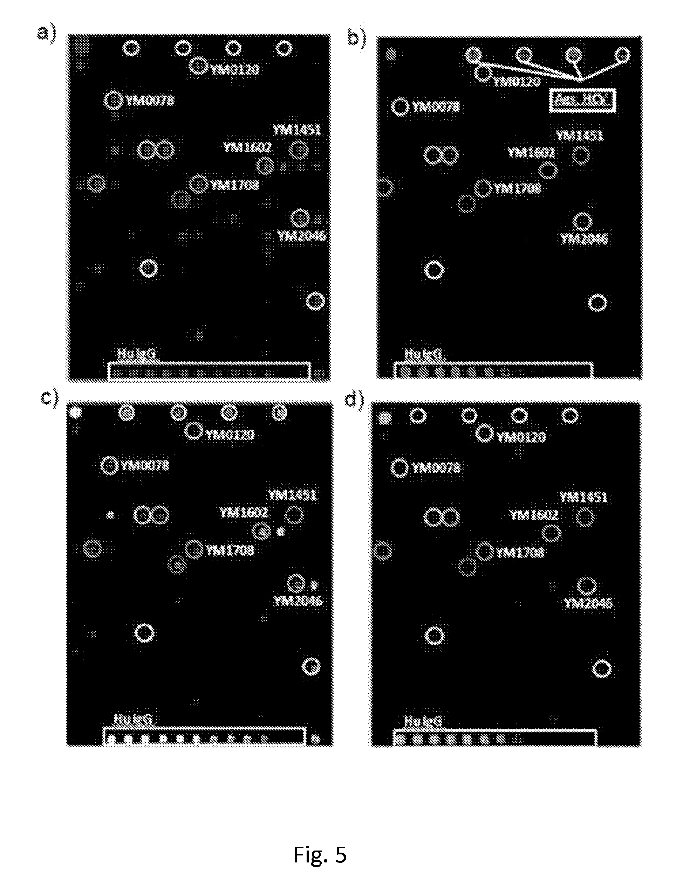

**Figure 5****:** Differences in immunoreactivity between serum samples. Representative image of the autoantigen microarrays incubated with patient's serum from **a)** autoimmune liver disease (Al), **b)** HCV **c)** HCV with autoimmunity extrahepatic (HCV+Al) and **d)** healthy donor (HD) were compared.

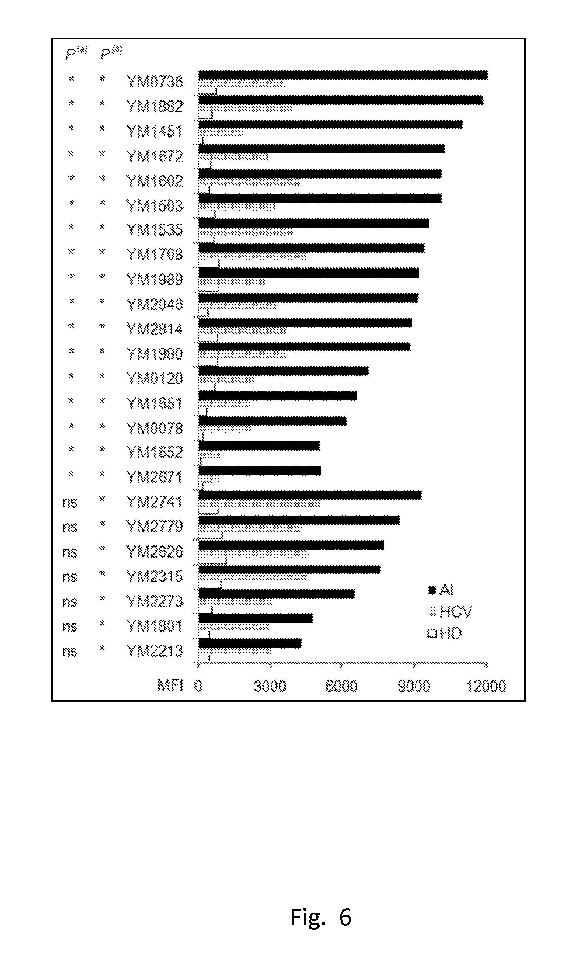

**Figure 6****:** Mean Fluorescence intensities of the 24 autoantigens selected in a whole Discovery sample set (training and test set). No significant differences were observed between the group of patients sera for seven antigens. Asterisks, p val<0.01 (Student's t-test). P^{(a)} val of Autoimmune patients *versus* Hepatitis C patients, P^{(b)} val of Autoimmune patients *versus* Healthy donors. HCV=Hepatitis C Virus (patients w/wo autoimmune disease); Al = autoimmune liver disease (patients with PBC and AIH disease); HD= Healthy Donors.

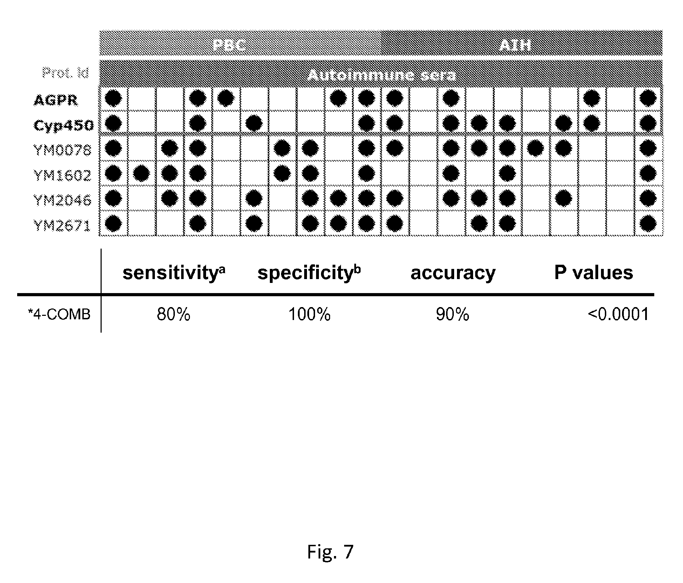

**Figure 7****:** Comparative sensitivity of the 4 antigens reported in Table 10 for autoimmune liver disease diagnosis. Sera are arranged along the columns, whereas top antigens are arranged along the rows. Each dot indicate a positive diagnosis. P values indicated in the bottom right result from the comparison of Mean Fluorescence Intensities between the autoimmune patients versus healthy donors, calculated with two-sided Fisher's exact test. *4-COMB: top 4 autoantigens in combination as diagnostic markers. AGPR = asialoglycoprotein receptor-1; CYP2D6= Cytochrome P450 2D6
   ^{(a)}Sensitivity is defined as % of positive autoimmune patients.
   ^{(b)}Specificity is defined as % of negative healthy donors.

Accuracy is expressed as percentage of true positives and negatives in the whole population.

### BRIEF DESCRIPTION OF THE HUMAN SEQUENCES LISTED

| **SEQ ID NO** | **Prot_ID** | **Accession Transcript** | **Amino acid sequence** |
|---|---|---|---|
| SEQ ID NO: 1 | YM0078 | 3566 | PRAPGNLTVHTNVSDTLLLTWSNPYPPDNYLYNHLTYAVN IWSENDPADFRIYNVTYLEPSLRIAASTLKSGISYRARVRA WAQCYNTTWSEWSPST |
| SEQ ID NO: 2 | YM0120 | *129530* | SNWGCYGNIQSLDTPGASCGIGRRHGLNYCGVRASERLA EIDMPYLLKYQPMMQTIGQKYCMDPAVIAGVLSRKSPGDK ILVNMGDRTSMVQDPGSQAPTSWISESQVSQTTEVLTTRI KEIQRRFPTWTPDQYLRGGLCAYSGGAGYVRSSQDLSCD FCNDVLARAKYLKRHGF |
| SEQ ID NO: 3 | YM0736 | *126526* | MFVDNRIQKSMLLDLNKEIMNELGVTVVGDIIAILKHAKVVH RQDMCKAATESVPCSPSPLAGEIRRGTSAASRMITNSLNH DSPPSTPPRRPDTSTSKISVTVSNKMAAKSAKATAALARR EEESLAVPAKRRRVTAEMEGKYVINMPKGTTPRTRKILEQ QQAAKGLHRTSVFDRLGAETKADTTTGSKPTGVFSRLGA TPETDEDLAWDSDNDSSSSVLQYAGVLKKLGRGPAK |
| SEQ ID NO: 4 | YM1414 | *MM_030652* | SQGVCSKQTLVVPLHYNESYSQPVYKPYLTLCAGRRICST YRTMYRVMWREVRREVQQTHAVCCQGWKKRHPGALTC EA |
| SEQ ID NO: 5 | YM1451 | *400943* | HEAHKTSLSSWKHDQDWANVSNMTFSNGKLRVKGIYYR NADICSRHRVTSAGLTLQDLQLWCNLRSVARGQIPSTL |
| SEQ ID NO: 6 | YM1503 | *399716* | GVAEFHMSLTVSCPDPTPSTDPQGRHNREPILGRDDDFM CKQVKFRMCVVGRDGNAQSSVRYTGPLYRRKIRTEFLFV VFLLETRELKPQVNKNVQGTRPS |
| SEQ ID NO: 7 | YM1535 | *114991* | |
| SEQ ID NO: 8 | YM1602 | *646100* | ATGILICMTKNLESVHSIVLAHSCYHHENKPRPDCCFQQKI RDTKSKVELPRHAHARLTNPQLTHRSMKINDCCIKPLRFG VTCYAAFCDNN |
| SEQ ID NO: 9 | YM1651 | *100141515* | AREEEITPVVSIAYKVLEVFPKGRWVLITCCAPQPPPPITYS LCGTKNIKVAKKVVKTHEPASFNLNVTLKSSPDLLTYFCW ASSTSGAHVDSARLQMHWELWSKPVSELRANFTLQDRG AGPRVEMICQASSGSPPITNSLIGKDGQVHLQQRPCHRQ PANFSFLPSQTSDWFWCQAANNANVQHSALTVVPPGGD QKMEDWQGPLESPILALPLYRSTRRLSEEEFGGFRIGNGE |
| SEQ ID NO: 10 | YM1652 | *100141515* | VAKKVVKTHEPASFNLNVTLKSSPDLLTYFCWASSTSGAH VDSARLQMHWELWSKPVSELRANFTLQDRGAGPRVEMI CQASSGSPPITNSLIGKDGQVHLQQRPCHRQPANFSFLPS QTSDWF |
| SEQ ID NO: 11 | YM1672 | *284207* | AQYSSDRCSWKGSGLTHEAHRKEVEQVYLRCAAGAVEW MYPTGALIVNLRPNTFSPARHLTVCIRSFTDSSGANIYLEK TGELRLLVPDGDGRPGRVQCFGLEQGGLFVEATPQQDIG RRTTGFQYELVRRHRASDLHELSAPCRPCSDTEVLLAVCT |
| | | | SDFAVRGSIQQVTHEPERQDSAIHLRVSRLYRQKSRVFEP VPEGDGHWQGRVRTLLECGVRPGHGDFLFTGHMHFGEA R |
| SEQ ID NO: 12 | YM1708 | *ENSP00000 392824* | MFGVLEGAQANSENWIAPSGPWALGLWSSLYFLLFSTLE GRGGRVLSQSCSMAVAAASWISRENARSVKRSYMQSSP QRPKEPRNQRTSHTTPVC |
| SEQ ID NO: 13 | YM1801 | *134548* | SGFTALHWAAKSGDGEMALQLVEVARRSGAPVDVNARS HGGYTPLHLAALHGHEDAAVLLVVRLGAQVHVRDHSGRR AYQYLRPGSSYALRRLLGDPGLRGTTEPDATGGGSGSLA ARRPVQVAATILSSTTSAFLGVLADDLMLQDLARGLKKSS SFSKFLSASPMAPRKKTKIRGGLPAFSEISRRPTPGPLAGL VPSFPPTT |
| SEQ ID NO: 14 | YM1882 | *119587* | SLIVFMEQVHRGIKGLVRDSHGKGIPNAIISVEGINHDIRTA NDGDYWRLLNPGEYVVTAKAEGFTASTKNCMVGYDMGA TRCDFTLSKTNMARIREIMEKFGKQPVSLPARRLKLRGQK RRQRG |
| SEQ ID NO: 15 | YM1980 | *340204* | GSLSPTKYNLLELKESCIRNQDCETGCCQRAPDNCESHC AEKGSEGSLCQTQVFFGQYRACPCLRNLTCIYSKNEKWL SIAYGRCQKIGRQKLAKKMFF |
| SEQ ID NO: 16 | YM1989 | *150356* | SQLELIDLSSNPFHCDCQLLPLHRWLTGLNLRVGATCATP PNARGQRVKAAAAVFEDCPGWAARKAKRTPASRPSARR TPIKGRQCGADKVGHGAGGV |
| SEQ ID NO: 17 | YM2046 | *164284* | GEAGGSCLRWEPHCQQPLPDRVPSTAILPPRLNGPWIST GCEVRPGPEFLTRAYTFYPSRLFRAHQFYYEDPFCGEPA HSLLVKGKVRLRRASWVTRGATEADYHLHKVGIV |
| SEQ ID NO: 18 | YM2213 | *ENSP00000 414589* | ATKLVTCPAPRQFAVGAFTAAGRAWLFAPSLGASFSKLRS QQRSRDFRGRLFLRAERRAGGFTS |
| SEQ ID NO: 19 | YM2273 | *ENSP00000 374868* | SSNLEGRTKSVIRQTGSSAEITCDLAEGSTGYIHWYLHQE GKAPQRLLYYDSYTSSVVLESGISPGKYDTYGSTRKNLRM ILRNLIENDSGVYYCATWDGHSDSDPPYTTLKTCLVAASP REEGMRWALLVLLAFLSPIPAPPTVFCARDHFLVEWDLSF ERIFYSFSSGPFPSKAPERKACSQKSSNLEGRMKSVTRPT GSSAEITCDLTVINAVYIHWYLQQEGKTPQHLLHYDV |
| SEQ ID NO: 20 | YM2279 | *AAC13327* | AGVTQTPKFHVLKTGQSMTLLCAQDMNHEYMYRYRQDP GKGLRLIYYSVAAALTDKGEVPNGYNVSRSNTEDFPLKLE SAAPSQTSVYFCASSYSTALQGCLLSAHKGKGRCCPPPP PKTQGCPVQRSLHQEPWNPEWPQVARTV |
| SEQ ID NO: 21 | YM2315 | *ENSP00000 374919* | AKVTQTPGHLVKGKGQKTKMDCTPEKGHTFVYWYQQNQ NKEFMLLISFQNEQVLQETEMHKKRFSSQCPKNAPCSLAI LSSEPGDTALYLCASSQSTALKCQFLLAHKLVTDPAQEAG DVLGWKGVTENNWSQLKPQCNLTQG |
| SEQ ID NO: 22 | YM2671 | *280664* | RDKMRMQRIKVCEKRPSIDLCIHHCSYFQKCETNKICCSA FCGNICMSIL |
| SEQ ID NO: 23 | YM2779 | *57828* | QLLMYQQHTSHYDLERKGGYLMLSFIDFCPFSVMRLRSLP SPQRYTRQERYRARPPRVLERSGFHNENSLAIYQGLVYY LLWLHSVYDKPYADPVHDPTWRWWANNKQDQDYYFFLA SNWRSAGGVSIEMDSYEKIYNLESAYELPERIFLDKGTEY SFAIFLSAQGHSFRTQSELGTAFQLHSQVDVGVVLADPGC IEASVKQEVLINRNSVLFSITLKDKKLCYDQGISGHHLME |
| SEQ ID NO: 24 | YM2814 | *1101* | VVEELKLSHNPLKSIPDNAFQSFGRYLETLWLDNTNLEKF SDGAFLGVTTLKHVHLENNRLNQLPSNFPFDSLETLALTN NPWKCTCQLRGLRRWLEAKASRPDATCASPAKFKGQHIR DTDAFRSCKFPTKRSKKAGRH |

### DETAILED DESCRIPTION OF THE INVENTION

### Material and Methods

### Serum Samples

Samples used were collected by individuals from four different hospitals: i) Policlinico Ospedale Maggiore, Centro Trasfusionale, Milan; ii) UO Epatologia, Azienda Ospedaliero-Universitaria Pisana, Cisanello, Pisa; iii) University of Bologna. Policlinico Sant'Orsola-Malpighi, Bologna, Italy; iiii) Center for Systemic Manifestations of Hepatitis Viruses (MaSVE), Università Firenze.

Table 1 reports the clinical characteristics and ages of the patients and donors enrolled in this study, for the microarray analysis (Example 2-3).

**Table 1: Clinical characteristics of the samples used for serum profiling**

| **Group** | **Sub group** | **#** | **Source*** | **Age** | **Sex** |
|---|---|---|---|---|---|
| **Training Set** | | **115** | | ***Mean* ± SD *(median)*** | **Gen (#)** |
| Healthy Donors | | 39 | **1** | 44 ± 10 (46) | f(12) m(27) |
| HCV patients | with autoimmune disorders** | 13 | **2, 3** | 63 ± 13 (66) | f(9) m(4) |
| | with autoreactivity^{$} | 15 | **2, 3** | 53 ± 15 (59) | f(5) m(10) |
| | | 33 | **2, 3** | 56 ± 16 (57) | f(12) m(21) |
| Hepatic Autoimmunity patients | Autoimmune hepatitis | 8 | **3** | 53 ± 20 (52) | f(8) m(0) |
| | Primary Billiary Cirrhosis | 7 | **3** | 50 ± 12 (52) | f(6) m(1) |

| **Test Set** | | **115** | | | |
|---|---|---|---|---|---|
| Healthy Donors | | 39 | **1** | 45 ± 10 (45) | f(8) m(31) |
| HCV patients | with autoimmune disorders** | 13 | **2, 3** | 53 ± 14 (46) | f(6) m(7) |
| | with autoreactivity^{$} | 15 | **2, 3** | 57 ± 12 (59) | f(6) m(9) |
| | | 33 | **2, 3** | 50 ± 14 (46) | f(8) m(24) |
| Hepatic Autoimmunity patients | Autoimmune hepatitis | 7 | **3** | 59 ± 22 (56) | f(5) m(2) |
| | Primary Billiary Cirrhosis | 8 | **3** | 51 ± 23 (54) | f(8) m(0) |

| | | | | | |
|---|---|---|---|---|---|
| * Origin of samples: **(1)** Policlinico Ospedale Maggiore, Centro Trasfusionale, Milan, **(2)** UO Epatologia, Azienda Ospedaliero-Universitaria Pisana, Cisanello, Pisa, **(3)** University of Bologna. Policlinico Sant'Orsola-Malpighi, Bologna, Italy. **Autoimmune disorders in patients with HC included Crioglobulinemia and Thyroid disfunctions; ^{$}patients with autoreactivity were defined as patients positive for Non-Organ-Specific-Autoantibodies (NOSA) (ANA, LKM, SMA, anti-DNA). | | | | | |

Table 2 reports the clinical characteristics of the patients and donors enrolled in this study, not used before, for the DELFIA^{®} assays (Example 4).

**Table 2: Clinical characteristics of the samples used for validation**

| **Group** | **Sub group** | **#** | **Source*** |
|---|---|---|---|
| **Validation Set** | | **70** | |
| Healthy Donors | | 20 | **1** |
| HCV patients | with autoimmune disorder** | 10 | **2** |
| | | 10 | **2** |
| Hepatic Autoimmunity patients | Autoimmune hepatitis | 10 | **3** |
| | Primary Billiary Cirrhosis | 10 | **3** |
| HBV patients | | 10 | **3** |

| | | | |
|---|---|---|---|
| *Origin of samples: **(1)** Policlinico Ospedale Maggiore, Centro Trasfusionale, Milan, **(2)** Center for Systemic Manifestations of Hepatitis Viruses (MaSVE), Università Firenze, **(3)** University of Bologna. Policlinico Sant'Orsola-Malpighi, Bologna, Italy. **Autoimmune disorders in patients with Hepatitis C included Crioglobulinemia. | | | |

### Human proteins: selection, expression and purification

With the goal of identifying novel diagnostic/prognostic candidate markers starting from a genome wide approach, genes whose translated products carry a secretion signal peptide or at least one transmembrane domain were selected, cloned and expressed in a high through-put system. A relevant portion of proteins included in this group belongs to "uncharacterized" proteins, i.e proteins for which a biological function has not yet been assigned. After expression in *E. coli* and purification from the bacterial insoluble fraction by Immobilized metal ion affinity chromatography (IMAC), 1658 full-length proteins or protein domains were successfully obtained as denatured recombinant products. A set of 75 well characterized membrane proteins putatively involved in autoimmune diseases were included, as well. As controls, human, viral or bacterial selected proteins were spotted on the microarray to be used as biological or technical controls. In particular genes encoding for Core protein and Non-structural proteins NS3 (from HCV genotype 1), NS3-4a (from HCV genotype 2) NS5b (from HCV genotype 1), Tetanus toxin and H1N1 were subcloned in *E. coli* strain DH5α and expressed in BL21(DE3), respectively. Genes encoding Cytochrome P450 2D6 (CYP2D6) and asialoglycoprotein receptor 1 (ASGR-1) from Ultimate™ Human ORF Clones were purchased by Invitrogen and were subcloned in *E. coli* strain DH5α and expressed in BL21 (DE3), respectively. All the corresponding proteins were purified by affinity chromatography on IMAC resin.

### Protein quality control

Purified recombinant proteins (10-15 µg total protein), obtained as described above, were stored at 4°C and analyzed by SDS-PAGE (Criterion PAGE system Bio-Rad) follow by Coomassie Blue staining of the gel immediately before spotting them, to assess their integrity, identity, and purity level. The identity of 93% of the purified proteins was confirmed based on their expected molecular weights. Moreover, 90% of the spotted proteins displayed purity levels >70% (ChemiDoC^{™} XRS, Quantity One^{®} software; Bio-Rad).

For Western blot analysis, aliquots (0.5 µg) of the proteins were resolved on 4-12% pre-cast SDS-PAGE gradient Tricine gels under reducing conditions, and electroblotted onto nitrocellulose membranes (Bio-Rad), according to the manufacturer's instructions. The membranes were blocked with 5% non-fat milk in 1x PBS plus 0.1% Tween 20 (PBST) for 1 h at room temperature, incubated with the α-His mAb (GE-Healthcare) diluted 1:1000 in 3% non-fat milk in PBST 0.1% for 1 h at room temperature, and washed three times in PBST 0.1% (Fig. 1). The secondary HRP-conjugated antibody (α-mouse immunoglobulin/HRP, GE-Healthcare) was diluted 1:1000 in non-fat milk in PBST-0.1% and incubated for 1 h at room temperature. The proteins were visualized by enhanced chemiluminescence applying Super Signal West Pico Chemiluminescence Substrate (Thermo Scientific, USA) according to the manufacturer's specifications. Chemiluminescence was detected with an LAS-3000 (Fujifilm, USA).

### Protein microarray printing

A Human Protein MicroArray was generated by spotting the 1658 affinity-purified recombinant proteins (0.5 mg/ml, in Urea 6M) in 4 replicates on nitrocellulose-coated slides (FAST slides, GE-Healthcare) using Stealth SMP3^{®} spotting pins (TeleChem International, Sunnyvale, California) and a Microgrid II microarray contact printer (Biorobotics), resulting in spots of approximately 130 µm in diameter. As experimental positive control, to assess the sensitivity and reproducibility of the arrays and for signal normalization, a curve of human IgG(s) (solutions from 0.001 to 1 mg/ml) was spotted on the arrays in 8 replicates (in Urea 6M) and detected with Alexa-647 conjugated α-Human IgG secondary antibody (Invitrogen). As negative controls the spotting buffer alone, was printed and used to assess possible non-specific signals due to cross contamination. A quality control of the spotting procedure was performed on 10% of randomly chosen slides, by confirming the presence of the total immobilized proteins using the α-His mAb, followed by detection using a Alexa-647 conjugated α-Human IgG secondary antibody (Fig. 2). The spotted microarrays were allowed to remain at room temperature for 1 h before storage at 4 °C until use.

### Incubation and scanning of protein microarray

Incubation was automatically performed with a TECAN Hybridization Station (HS 4800™ Pro; TECAN, Salzburg, Austria). The microarray slides were prewashed 3 min in PBST 0.1% Tween 20, and saturated with BlockIt™ Microarray Blocking Buffer (Arrayit Corporation) for 45 min at 25°C under mild agitation. After injection of 105 µl of individual serum diluted 1:300 in Blocking Buffer plus 0.1 % Tween 20, incubation was performed at 25°C for 45 min with low agitation. The microarrays were washed at 25°C in PBST for three cycles of 1 min wash time and 30 sec soak time.

Afterwards the microarray slides were incubated at 25°C for 1 with Alexa-647 conjugated α-human IgG (Invitrogen) diluted at 1:800 in Blocking Buffer in the dark. The microarrays were again washed at 25°C in PBST for two cycles of 1 min wash time and 30 sec soak time, in PBS for two cycles of 1 min: 30 sec and finally in milliQ sterile water for one cycle of 15 sec.

The microarray slides were finally dried at 30°C under nitrogen for 2 min, and scanned using a ScanArray Gx PLUS (PerkinElmer, Bridgeport Avenue Shelton, USA). 16-bit images were generated with ScanArrayTM software at 10µm per pixel resolution and processed using ImaGene 8.0 software (Biodiscovery Inc, CA, USA). Laser of 635 nm was used to excite Alexa-647 dye. The fluorescence intensity of each spot was measured, signal-to-local-background ratios were calculated by ImaGene, and spot morphology and deviation from the expected spot position were considered using the default ImaGene settings.

### Data analysis

### Mean Fluorescence Intensity (MFI)

For each sample, the Mean Fluorescence Intensity (MFI) of replicated spots was determined, after subtraction of the background value for each spot, and subsequently normalized on the basis of the human IgG curve to allow comparison of data from different set of experiments (Bombaci M et al., PLoS One, 2009). Briefly, the MFIs values of IgG, spotted at different concentrations, were best fitted by a sigmoid curve, using a maximum likelihood estimator (Harris JW *et al.,* 1998). The experimental average IgG curve of each slide was adjusted on the reference sigmoid IgG curve, and the background-subtracted MFI values of each protein were normalized accordingly. On the basis of these results, a normalized MFI value of 4.000 was chosen as the lowest signal threshold for scoring a protein as positively recognized by human sera. For each protein, a Coefficient of Variation (CV%), was calculated on the four replicate spots, for intra-assay reproducibility (Bombaci et al., PLoS One, 2009).

### Recognition Frequency (RecFreq)

Percentage of sera reacting with a particular antigen in protein chip with a MFI ≥ 4.000. This threshold was defined as reported above. Moreover, to pass further analyses, an antigen must have a CV% correlated to its MFI on the basis of standard IgG curves. If the CV% value is not within this range the data cannot be considered reliable, therefore the antigen is discarded being defined as "absent" from the slide.

In order to get a profile of the specificity of the immunologic responses for each serum and to compare the antigen recognition patterns among the different groups of sera, the normalized MFI values were subjected to an unsupervised bi-dimensional hierarchical clustering, using TIGR Multiexperiment Viewer (version MeV4.5) software.

### DELFIA^{®} assays

The Dissociation-Enhanced Lanthanide Fluoroscence ImmunoAssay method (DELFIA^{®}) is a time-resolved fluorescence method that can be used to study antibody binding to solid-phase proteins or peptides. The purified recombinant proteins were used at a concentration of 20 µg per milliliter (as in Frulloni L et al., N Engl J Med. 2009) in urea 6 M to coat DELFIA^{®} plates (PerkinElmer). Plates were then blocked for 1 hour at 37°C with a blocking reagent (PerkinElmer). The blocking buffer was than discarded, and the serum samples were diluted in a 1:300 solution in phosphate buffered saline plus 1% bovine serum albumin (Sigma), plus 0.1 %Tween 20 (Sigma) and incubated on the plates 1 hour at 37°C. Plates were then washed 5 times with washing buffer (PerkinElmer). Bound antibodies were detected with europium-labeled α-human IgG serum (1:500 in diluting buffer, PerkinElmer), incubated 30 min at RT in the dark. The wells were again washed in the same washing buffer. After a 10 min incubation at RT, the plates were read on a Infinite F200 PRO instrument (Tecan). Fluorescence intensity values higher than the mean plus 3 standard deviations for each serum in the Healhy donor's group were considered to be positive.

### Statistical analysis

The performance of the identified signatures was evaluated by statistical analysis. In particular results of DELFIA^{®} and Protein Microarray experiments from sera of patients and healthy donors were compared using the two-tailed χ², the Student's t-test or the Fisher's exact tests. The ANOVA test was used for all the others analyses. Statistical analysis was carried out with the use of GraphPad Prism 5 software, version 5.01.

### EXAMPLES

### Example 1- Design and Development of a Human Protein Microarray

To study the serological profile of patients diagnosed with autoimmune liver diseases versus a panel of self proteins, the authors developed a protein array by printing 1658 human recombinant proteins (see details in *Materials and Methods* section) distributed as shown in Table 3.

**Table 3: Compartment sub-cellular distribution of the human proteins spotted.**

| **Compartment** | **n. of proteins** |
|---|---|
| Full length, secreted protein | 393 |
| Secreted Domain | 150 |
| Extracellular Domain | 498 |
| Domain | 226 |
| Extracellular Domain plus TMs/cytosolic | 59 |
| region | |
| Full length - transmembrane | 145 |
| Cytosolic protein | 4 |
| Others | 183 |
| | |
| *Total Proteins* | *1658* |

Briefly, the proteins were first selected through a bioinformatic analysis of the whole human genome as translated sequences carrying i) signal peptides, ii) at least one transmembrane domain, iii) having unknown biological function. The majority of printed human proteins were expressed as N-terminal His-tag fusions while 48 proteins where expressed as double tagged fusions, with an N-terminal glutathione S-transferase (GST) and with C-terminal Histidines. Proteins obtained after affinity purification from the bacterial insoluble fraction showed purity levels > 70%, as estimated by densitometric scan of SDS-PAGE gels (see *Materials and Methods*). Moreover, we performed western blot analysis on random protein samples with the anti-His mAb. About 80% of the probed proteins gave a positive signal. In addition for almost 90% of the latter proteins, the molecular weights of the bands detected by western blot corresponded to those observed by SDS-PAGE (Fig. 1).

The list of proteins printed on the array includes well-known virulence factors such as 5 HCV structural and non structural proteins (see *Materials and Methods*), the tetanus toxoid, influenza antigen H1N1 and 4 immunogenic bacterial proteins, used as positive controls. In addition, staphylococcal protein A and anti-human IgG antibodies were spotted as technical positive controls for grid orientation and to check the hybridization quality. As negative controls, 3 proteins such as human serum albumin (HSA), bovine serum albumin (BSA) and human Glutathione-S-Transferase (hu-GST) were used (*Materials and Methods*)*.* Each protein was printed in 4 replicates, spatially distributed in a randomized manner to guarantee the maximum reliability of the signal reproducibility. The final protein array design consisted of 24 grids each of 304 spots, for a total of 7296 spots (Fig. 2a).

The quality and quantity of the immobilized proteins on the chips were determined by probing with an anti-His mAb, and 89% of the proteins produced signals that were significantly above the background (Fig. 2b). Eleven different amounts of human IgGs at a known concentration (from 8.24x10⁻⁴ to 7x10⁻¹ ng of immobilized protein/spot) were printed also on the array in eight replicates. They were used as technical controls to set up the detection parameters, for data normalization and to test the reproducibility of the system (Fig. 2c). To compare data from different experiments, we used a normalization method, as previously described (Bombaci M et al., PLoS One, 2009). Briefly, the experimental average IgG curve of each slide was adjusted on a reference sigmoid IgG curve, and the background-subtracted mean fluorescence intensities (MFIs) values of each protein were normalized accordingly. On the basis of these results, a normalized-MFI value of 4000 was chosen as the lowest signal threshold for scoring a protein as positively recognized by human sera. The correlation among spot intensities of two different slides of the same batch indicates a high reproducibility of the signals derived from the proteins spotted (Fig. 2d).

### Example 2- Patients affected by autoimmune liver diseases show higher auto-immunoreactivity as compared to healthy donors

In an attempt to determine if the panel of proteins described above was recognized by patients sera with autoimmune liver disease, the protein microarray was probed with a total of 115 serum samples (see training set as indicated in Table 1 in *Materials and Methods*) from 15 patients with autoimmune liver diseases, 61 patients with hepatitis C and 39 sera from healthy donors. In more details, the authors analyzed 33 patients with HCV infection, 13 patients with hepatitis C and extrahepatic autoimmune diseases, 15 patients with hepatitis C and autoreactivity, 8 patients with autoimmine hepatitis (AIH) and 7 patients with primary biliary cirrhosis (PBC). The clinical characteristics of each group of sera are summarized in Table 1.

Sera reactivity was evaluated by detecting total IgG bound to each protein spot using Alexa-647 conjugated α-human IgG and measuring the fluorescence intensity (FI) values for each protein.

As depicted in Figure 3a, the strongest autoreactivity was observed against all the proteins printed in patients sera either with (HCV patients) or without HCV infection (AI patients), but not in the control sera (HD group). Significant divergences between patients and healthy donors were also observed in terms of the recognition frequencies, indicating differential protein-specific IgG levels: in details, the percentage of proteins recognized by more than 15% of the tested sera with MFI>4000 was about 8% (132 proteins) in the case of both patients groups but only 3% (41 proteins) in the case of healthy donors (Figure 3b). On the other hand, 53% of patients with liver autoimmunity and 39% of patients with HCV infection contain antibodies that react intensively with at least 3% of the proteins on the arrays, whereas only 18% of 39 controls had such antibodies (Figure 3c). In both cases the differences observed between the reactivity of patients and healthy donors were statistically significant (chi-square test, p val <0.01).

### Example 3- Selection and identification of specifically recognized autoantigens profile

Having previously established that both class patients (HCV and Al patients) of training set displayed an increased autoreactivity when compared to healthy donors, the authors selected the autoantigens specifically recognized by those patients. Figure 4 shows the flowchart describing the discovery and validation phases performed in the present invention. Following normalization, individual autoantigens from protein microarray were ranked according to:
i) the recognition frequency of patients from the two groups of reactive sera (with autoimmune disease and hepatitis C, respectively) and
ii)the mean fluorescence intensities, as compared to the healthy donors.

To be considered of potential interest, the antigen-specific responses had to occur with a significantly higher frequency in patients sera than in healthy donors sera. In particular, the proteins should be recognized in less than 10% of the healthy donors sera and in at least 25% of sera from patients groups with a statistical significance having pval <0.01. By using this approach, the authors identified 24 distinct proteins showing a higher immunogenicity in patients with liver autoimmune diseases. These proteins allowed for distinguishing serum samples from patients diagnosed with autoimmune diseases from those of controls in the training set (Table 4).

**Table 4 Proteins recognized with higher frequency by patients compared to healthy donors in samples TrainingSet.**

| | | | n. Obs (%) | | | p val | |
|---|---|---|---|---|---|---|---|
| **Accession Transcript** | **Description** | **Protein Id** | **HD** | **HCV** | **Al** | **Al^{(a)}** | **HCV^{(b)}** |
| *3566* | interleukin 4 receptor | YM0078 | 0/29 | 8/50 | 7/15 | <0.01 | n.s. |
| | | | (0) | (16) | (47) | | |
| *129530* | lysozyme G-like 1 | YM0120 | 2/39 | 12/61 | 9/15 | <0.01 | n.s. |
| | | | (5) | (20) | (60) | | |
| *126526* | chromosome 19 open reading frame 47 | YM0736 | 1/29 | 14/48 | 10/15 | <0.01 | n.s. |
| | | | (3) | (29) | (67) | | |
| *MM_030652* | EGF-like-domain, multiple 8 | YM1414 | 0/39 | 3/61 | 6/15 | <0.01 | n.s. |
| | | | (0) | (5) | (40) | | |
| *400943* | AILT5830 | YM1451 | 1/36 | 7/54 | 8/15 | <0.01 | n.s. |
| | | | (30) | (13) | (53) | | |
| *399716* | hypothetical protein DKFZp667F0711 | YM1503 | 2/39 | 15/61 | 9/15 | <0.01 | n.s. |
| | | | (5) | (25) | (60) | | |
| *114991* | zinc finger protein 618 | YM1535 | 3/35 | 14/48 | 10/15 | <0.01 | n.s. |
| | | | (9) | (29) | (67) | | |
| *646100* | hypothetical protein XP_934154 | YM1602 | 1/39 | 17/61 | 9/15 | <0.01 | n.s. |
| | | | (3) | (28) | (60) | | |
| *100141515* | chromosome 17 open reading frame 99 | YM1651 | 0/39 | 8/61 | 8/15 | <0.01 | n.s. |
| | | | (0) | (13) | (53) | | |
| *100141515* | chromosome 17 open reading frame 99 | YM1652 | 0/35 | 4/51 | 7/15 | <0.01 | n.s. |
| | | | (0) | (8) | (47) | | |
| *284207* | meteorin, glial cell differentiation regulator-like | YM1672 | 2/39 | 13/61 | 8/15 | <0.01 | n.s. |
| | | | (5) | (21) | (53) | | |
| *ENSP00000392824* | LP5624 | YM1708 | 3/39 | 21/61 | 9/15 | <0.01 | n.s. |
| | | | (8) | (34) | (60) | | |
| *134548* | ankyrin repeat domain 43 | YM1801 | 0/39 | 14/61 | 6/15 | <0.01 | n.s. |
| | | | (0) | (23) | (40) | | |
| *119587* | carboxypeptidase X (M14 family), member 2 | YM1882 | 1/36 | 16/56 | 9/15 | <0.01 | n.s. |
| | | | (3) | (29) | (60) | | |
| *340204* | vitamin D (1,25- dihydroxyvitamin D3) receptor | YM1980 | 2/39 | 18/61 | 10/15 | <0.01 | n.s. |
| | | | (5) | (30) | (67) | | |
| *150356* | chondroadherin-like | YM1989 | 2/39 | 14/61 | 8/15 | <0.01 | n.s. |
| | | | (5) | (23) | (53) | | |
| *164284* | adenomatosis polyposis coli down- regulated 1-like | YM2046 | 1/35 | 10/48 | 8/15 | <0.01 | n.s. |
| | | | (3) | (21) | (53) | | |
| *ENSP00000414589* | | YM2213 | 0/35 | 13/61 | 7/15 | <0.01 | n.s. |
| | | | (0) | (21) | (47) | | |
| *ENSP00000374868* | | YM2273 | 0/39 | 13/61 | 7/15 | <0.01 | n.s. |
| | | | (0) | (21) | (47) | | |
| *AAC13327* | | YM2279 | 0/31 | 16/48 | 7/15 | <0.01 | n.s. |
| | | | (0) | (33) | (47) | | |
| *ENSP00000374919* | | YM2315 | 3/39 | 21/61 | 9/15 | <0.01 | n.s. |
| | | | (8) | (34) | (60) | | |
| *280664* | WAP four-disulfide core domain 10B | YM2671 | 0/39 | 3/61 | 6/15 | <0.01 | n.s. |
| | | | (0) | (5) | (40) | | |
| *57828* | neurofilament, | YM2779 | 3/39 | 16/61 | 10/15 | <0.01 | n.s. |
| | heavy polypeptide | | (8) | (26) | (67) | | |
| *1101* | chondroadherin | YM2814 | 2/39 | 16/61 | 9/15 | <0.01 | n.s. |
| | | | (5) | (26) | (60) | | |

| | | | | | | | |
|---|---|---|---|---|---|---|---|
| Statistics: Fisher exact test, FDR significance criterion, Benjamini-Hochberg correction, p val <0.01. (a)p val of Autoimmune patients versus Healthy donors. (b)p val of Hepatitis C patients versus Healthy donors. n.s.: not significant. | | | | | | | |

In order to confirm the above results, a different set of proteins spotted in the microarray was used. This microarray now included all proteins identified as more reactive in the training set (Table 5) including the 24 proteins as indicated above.

**Table 5: Proteins printed on the focused microarray.**

| **#** | **Accession Transcript** | **Description** | **Protein Array_Id** |
|---|---|---|---|
| 1 | *NM_000073* | CD3g molecule, gamma (CD3-TCR complex) | YM0066 |
| 2 | *BC042665* | CD80 molecule | YM0071 |
| 3 | *BC040261* | CD86 molecule | YM0072 |
| 4 | *BC007037* | CD69 molecule | YM0073 |
| 5 | *BC025691* | interleukin 2 receptor, beta | YM0076 |
| 6 | *3566* | interleukin 4 receptor | YM0078 |
| 7 | *BC015916* | integrin, beta 7 | YM0079 |
| 8 | *BC001188* | transferrin receptor (p90, CD71) | YM0083 |
| 9 | *BC010446* | chromosome 19 open reading frame 4 | YM0099 |
| 10 | *BC002525* | KIAA0494 | YM0100 |
| 11 | *BC008474* | chromosome 10 open reading frame 70 | YM0107 |
| 12 | *BC035671* | discoidin, CUB and LCCL domain containing 1 | YM0117 |
| 13 | *129530* | lysozyme G-like 1 | YM0120 |
| 14 | *BC020848* | ribonuclease, RNase A family, k6 | YM0125 |
| 15 | *BC029557* | hypothetical protein MGC39497 | YM0724 |
| 16 | *BC058929* | ubiquitin-like 4B | YM0728 |
| 17 | *126526* | chromosome 19 open reading frame 47 | YM0736 |
| 18 | *BC002748* | transmembrane protein 70 | YM0757 |
| 19 | *BC061899* | chromosome 14 open reading frame 49 | YM0838 |
| 20 | *BC006242* | hypothetical protein MGC11332 | YM0872 |
| 21 | *BC066910* | chromosome 6 open reading frame 68 | YM0887 |
| 22 | *BC022410* | chromosome 18 open reading frame 19 | YM0908 |
| 23 | *BC018995* | family with sequence similarity 70, member B | YM0972 |
| 24 | *BC012814* | zinc finger protein-like 1 | YM1020 |
| 25 | *BC028152* | CD33 molecule | YM1046 |
| 26 | *BC014601* | sushi domain containing 3 | YM1073 |
| 27 | *BC068556* | hypothetical protein LOC283537 | YM1077 |
| 28 | *BC074751* | chemokine (C-C motif) receptor 2 | YM1099 |
| 29 | *NM_153613* | 1-acylglycerol-3-phosphate O-acyltransferase 7 (lysophosphatidic acid acyltransferase, eta) | YM1129 |
| 30 | *NM_145231* | chromosome 14 open reading frame 143 | YM1131 |
| 31 | *BC085612* | | YM1134 |
| 32 | *BC063844* | family with sequence similarity 82, member C | YM1183 |
| 33 | *NM_014045* | low density lipoprotein receptor-related protein 10 | YM1204 |
| 34 | *NM_207303* | attractin-like 1 | YM1208 |
| 35 | *XM_371956* | KIAA1549 protein | YM1214 |
| 36 | *XM_290842* | leucine rich repeat and fibronectin type III domain containing 1 | YM1217 |
| 37 | *BC042951* | transcription elongation regulator 1-like | YM1340 |
| 38 | *XM_113796* | GRAM domain containing 2 | YM1342 |
| 39 | *XM_029962* | potassium channel, subfamily T, member 1 | YM1343 |
| 40 | *NM_021945* | chromosome 6 open reading frame 85 | YM1351 |
| 41 | *NM_178497* | hypothetical protein FLJ23657 | YM1401 |
| 42 | *MM_030652* | EGF-like-domain, multiple 8 | YM1414 |
| 43 | *XM_290972* | zinc and ring finger 3 | YM1423 |
| 44 | *400943* | AILT5830 | YM1451 |
| 45 | *NM_213602* | CD33 molecule-like 3 | YM1477 |
| 46 | *399716* | hypothetical protein DKFZp667F0711 | YM1503 |
| 47 | *BC020568* | chromosome 10 open reading frame 54 | YM1526 |
| 48 | *BC007412* | chromosome 14 open reading frame 153 | YM1529 |
| 49 | *BC093909* | hypothetical protein LOC284402 | YM1534 |
| 50 | *114991* | zinc finger protein 618 | YM1535 |
| 51 | *NM_001013403* | hypothetical LOC347487 | YM1536 |
| 52 | *BC033882* | chromosome 17 open reading frame 83 | YM1537 |
| 53 | *NM_001012278* | hypothetical protein LOC401507 | YM1542 |
| 54 | *XM_925796* | similar to Killer cell immunoglobulin-like receptor 3DL2 precursor (MHC class I NK cell receptor) (Natural killer associated transcript 4) (NKAT-4) (p70 natural killer cell receptor clone CL-5) | YM1546 |
| 55 | *NM_001039906* | hypothetical protein LOC644975 | YM1587 |
| 56 | *NM_001039795* | | YM1593 |
| 57 | *XM_940430* | hypothetical protein LOC648852 | YM1594 |
| 58 | *XM_927076* | hypothetical protein LOC643797 | YM1599 |
| 59 | *646100* | hypothetical protein XP_934154 | YM1602 |
| 60 | *100141515* | chromosome 17 open reading frame 99 | YM1651 |
| 61 | *100141515* | chromosome 17 open reading frame 99 | YM1652 |
| 62 | *XM_375430* | hypothetical protein LOC201229 | YM1668 |
| 63 | *BC031286* | chromosome 17 open reading frame 74 | YM1671 |
| 64 | *284207* | meteorin, glial cell differentiation regulator-like | YM1672 |
| 65 | *BC107110* | transmembrane protein 95 | YM1674 |
| 66 | *BC107110* | transmembrane protein 95 | YM1675 |
| 67 | *XM_928339* | similar to RIKEN cDNA 0610013E23 | YM1684 |
| 68 | *NM_145003* | t-SNARE domain containing 1 | YM1693 |
| 69 | *ENSP00000392824* | LP5624 | YM1708 |
| 70 | *NM_001012731* | hypothetical protein LOC283874 | YM1720 |
| 71 | *BC063012* | cysteine-rich secretory protein LCCL domain containing 2 | YM1770 |
| 72 | *BC044574* | enoyl Coenzyme A hydratase domain containing 2 | YM1778 |
| 73 | *134548* | ankyrin repeat domain 43 | YM1801 |
| 74 | *BC042877* | RPE-spondin | YM1810 |
| 75 | *OTTHUMT00000073980* | | YM1824 |
| 76 | *119587* | carboxypeptidase X (M14 family), member 2 | YM1882 |
| 77 | *BC031564* | chromosome 11 open reading frame 75 | YM1909 |
| 78 | *BC009510* | chromosome 9 open reading frame 123 | YM1914 |
| 79 | *BC005056* | Williams Beuren syndrome chromosome region 18 | YM1925 |
| 80 | *OTTHUMT00000072560* | | YM1957 |
| 81 | *OTTHUMT00000059789* | | YM1960 |
| 82 | *ENST00000328411* | | YM1971 |
| 83 | *340204* | vitamin D (1,25- dihydroxyvitamin D3) receptor | YM1980 |
| 84 | *NM_001011880* | secretory protein LOC497190 | YM1985 |
| 85 | *150356* | chondroadherin-like | YM1989 |
| 86 | *OTTHUMT00000074924* | | YM2013 |
| 87 | *ENST00000303432* | | YM2036 |
| 88 | *164284* | adenomatosis polyposis coli down-regulated 1-like | YM2046 |
| 89 | *ENST00000318969* | CDNA FLJ30430 fis, clone BRACE2008960 | YM2054 |
| 90 | *ENST00000345013* | Seven transmembrane helix receptor | YM2071 |
| 91 | *ENST00000375211* | HSAL5836 | YM2094 |
| 92 | *ENST00000377210* | CDNA FLJ45218 fis, clone BRCAN2019653 | YM2100 |
| 93 | *ENST00000340623* | CDNA FLJ90687 fis, clone PLACE1006093 | YM2110 |
| 94 | *ENST00000342204* | AALS9165. [Source:Uniprot/SPTREMBL;Acc:Q6UXU0] | YM2111 |
| 95 | *ENST00000343738* | | YM2136 |
| 96 | *ENST00000382204* | EPWW6493 | YM2140 |
| 97 | *ENST00000382854* | CDNA FLJ41135 fis, clone BRACE2028970 | YM2144 |
| 98 | *ENST00000383764* | similar to YPLR6490 (LOC389102), misc RNA | YM2151 |
| 99 | *OTTHUMT00000036919* | family with sequence similarity 75, member A3 | YM2168 |
| 100 | *ENST00000304627* | | YM2202 |
| 101 | *ENSP00000414589* | | YM2213 |
| 102 | *ENST00000361502* | | YM2214 |
| 103 | *OTTHUMT00000050157* | | YM2223 |
| 104 | *OTTHUMT00000059164* | | YM2237 |
| 105 | *OTTHUMT00000059740* | | YM2250 |
| 106 | *OTTHUMT00000059781* | | YM2251 |
| 107 | *OTTHUMT00000059906* | | YM2255 |
| 108 | *OTTHUMT00000075879* | | YM2271 |
| 109 | *ENSP00000374868* | | YM2273 |
| 110 | *OTTHUMT00000060058* | | YM2276 |
| 111 | *AAC13327* | | YM2279 |
| 112 | *OTTHUMT00000109520* | | YM2284 |
| 113 | *OTTHUMT00000132432* | | YM2286 |
| 114 | *OTTHUMT00000146120* | | YM2289 |
| 115 | *OTTHUMT00000146398* | | YM2297 |
| 116 | *ENSP00000374919* | | YM2315 |
| 117 | *OTTHUMT00000151031* | | YM2322 |
| 118 | *OTTHUMT00000151349* | | YM2326 |
| 119 | *OTTHUMT00000151671* | | YM2335 |
| 120 | *OTTHUMT00000151873* | | YM2355 |
| 121 | *OTTHUMT00000151915* | | YM2388 |
| 122 | *BC001278* | protease, serine, 23 | YM2441 |
| 123 | *BC045113* | leucine rich repeat transmembrane neuronal 1 | YM2448 |
| 124 | *BC048285* | thymic stromal co-transporter | YM2453 |
| 125 | *BC039863* | expressed in prostate and testis | YM2511 |
| 126 | *NM-002589* | BH-protocadherin (brain-heart) | YM2570 |
| 127 | *NM_012285* | potassium voltage-gated channel, subfamily H (eag-related), member 4 | YM2576 |
| 128 | *BC005161* | inhibin, beta E | YM2613 |
| 129 | *BC058841* | ADAM metallopeptidase with thrombospondin type 1 motif, 12 | YM2616 |
| 130 | *BC074960* | ribonuclease, RNase A family, 7 | YM2626 |
| 131 | *NM_144701* | interleukin 23 receptor | YM2646 |
| 132 | *NM_130896* | WAP four-disulfide core domain 8 | YM2665 |
| 133 | *280664* | WAP four-disulfide core domain 10B | YM2671 |
| 134 | *BC074826* | cripto, FRL-1, cryptic family 1 | YM2707 |
| 135 | *BC022367* | R-spondin 3 homolog (Xenopus laevis) | YM2726 |
| 136 | *BC011057* | leucine rich repeat neuronal 6A | YM2728 |
| 137 | *BC034015* | progestin and adipoQ receptor family member VII | YM2741 |
| 138 | *BC070079* | leucine rich repeat containing 32 | YM2767 |
| 139 | *57828* | neurofilament, heavy polypeptide | YM2779 |
| 140 | *1101* | chondroadherin | YM2814 |
| 141 | *BC027883* | left-right determination factor 1 | YM2826 |
| 142 | *BC025316* | phospholipase A2, group III | YM2831 |
| 143 | *BC029864* | hyaluronan and proteoglycan link protein 2 | YM2843 |
| 144 | *BC069565* | interleukin 17E | YM2854 |

In order to determine which candidate autoantigens were autoimmune disease specific, the authors probed the focused microarray with a second sample set (Test Set as indicated in Table 1 *Material and Methods*) of 115 serum samples. In particular, sera of 33 patients with HCV infection were analyzed, of 13 patients with hepatitis C and extra-hepatic autoimmune diseases, of 15 patients with hepatitis C and autoreactivity, of 7 AIH and of 8 PBC patients. As negative controls, the authors also included 39 serum samples from healthy subjects. Representative probing images are shown in Figure 5a-d. Following the same normalization procedures (see *Materials and Methods),* the authors scored the reactivity on microarrays by comparing patients sera with those of the healthy donors (Table 6).

**Table 6: Proteins recognized with higher frequency by patients compared to healthy donors in sample Test Set.**

| | | | n. Obs (%) | | | p val | |
|---|---|---|---|---|---|---|---|
| **Accession Transcript** | **description** | **Protein ld** | **HD** | **HCV** | **Al** | **Al^{(a)}** | **HCV^{(b)}** |
| *3566* | interleukin 4 receptor | YM0078 | 0/27 | 8/46 | 9/15 | <0,0 | n.s. |
| | | | (0) | (17) | (60) | 1 | |
| *129530* | lysozyme G-like 1 | YM0120 | 0/39 | 9/61 | 10/15 | <0,0 | n.s. |
| | | | (0) | (15) | (67) | 1 | |
| *126526* | chromosome 19 open reading frame 47 | YM0736 | 1/27 | 17/45 | 9/15 | <0,0 | n.s. |
| | | | (4) | (38) | (60) | 1 | |
| *MM_030652* | EGF-like-domain, multiple 8 | YM1414 | 0/39 | 1/61 | 5/15 | n.s. | n.s. |
| | | | (0) | (2) | (33) | | |
| *400943* | AILT5830 | YM1451 | 0/34 | 10/50 | 12/15 | <0,0 | n.s. |
| | | | (0) | (20) | (80) | 1 | |
| *399716* | hypothetical protein DKFZp667F0711 | YM1503 | 0/39 | 18/61 | 11/15 | <0,0 | <0,01 |
| | | | (0) | (30) | (73) | 1 | |
| *114991* | zinc finger protein 618 | YM1535 | 0/34 | 13/39 | 11/15 | <0,0 | <0,01 |
| | | | (0) | (33) | (73) | 1 | |
| *646100* | hypothetical protein XP_934154 | YM1602 | 0/39 | 15/61 | 11/15 | <0,0 | <0,01 |
| | | | (0) | (25) | (73) | 1 | |
| *100141515* | chromosome 17 open reading frame 99 | YM1651 | 0/39 | 10/61 | 9/15 | <0,0 | n.s. |
| | | | (0) | (16) | (60) | 1 | |
| *100141515* | chromosome 17 open reading frame 99 | YM1652 | 0/34 | 4/46 | 7/15 | <0,0 | n.s. |
| | | | (0) | (9) | (47) | 1 | |
| *284207* | meteorin, glial cell differentiation regulator-like | YM1672 | 1/39 | 13/61 | 11/15 | <0,0 | n.s. |
| | | | (3) | (21) | (73) | 1 | |
| *ENSP00000392824* | LP5624 | YM1708 | 2/39 | 15/61 | 11/15 | <0,0 | n.s. |
| | | | (5) | (25) | (73) | 1 | |
| *134548* | ankyrin repeat domain 43 | YM1801 | 0/39 | 15/61 | 6/15 | <0,0 | <0,01 |
| | | | (0) | (25) | (40) | 1 | |
| *119587* | carboxypeptidase X (M14 family), member 2 | YM1882 | 0/34 | 16/50 | 12/15 | <0,0 | <0,01 |
| | | | (0) | (32) | (80) | 1 | |
| *340204* | vitamin D (1,25-dihydroxyvitamin D3) receptor | YM1980 | 1/39 | 17/61 | 10/15 | <0,0 | n.s. |
| | | | (3) | (28) | (67) | 1 | |
| *150356* | chondroadherin-like | YM1989 | 0/39 | 15/61 | 9/15 | <0,0 | <0,01 |
| | | | (0) | (25) | (60) | 1 | |
| *164284* | adenomatosis polyposis coli down-regulated 1-like | YM2046 | 0/34 | 11/39 | 11/15 | <0,0 | n.s. |
| | | | (0) | (28) | (73) | 1 | |
| *ENSP00000414589* | | YM2213 | 0/36 | 14/61 | 6/15 | <0,0 | n.s. |
| | | | (0) | (23) | (40) | 1 | |
| *ENSP00000374868* | | YM2273 | 1/39 | 15/61 | 11/15 | <0,0 | n.s. |
| | | | (3) | (25) | (73) | 1 | |
| *AAC13327* | | YM2279 | 0/31 | 13/39 | 6/15 | n.s. | <0,01 |
| | | | (0) | (33) | (40) | | |
| *ENSPOO000374919* | | YM2315 | 2/39 | 21/61 | 9/15 | <0,0 | |
| | | | (5) | (34) | (60) | 1 | n.s. |
| *280664* | WAP four-disulfide core domain 10B | YM2671 | 0/39 | 4/61 | 7/15 | <0,0 | n.s. |
| | | | (0) | (7) | (47) | 1 | |
| *57828* | neurofilament, heavy polypeptide | YM2779 | 2/39 | 18/61 | 11/15 | <0,0 | |
| | | | (5) | (30) | (73) | 1 | n.s. |
| *1101* | chondroadherin | YM2814 | 2/39 | 16/61 | 11/15 | <0,0 | n.s. |
| | | | (5) | (26) | (73) | 1 | |

| | | | | | | | |
|---|---|---|---|---|---|---|---|
| Statistics: Fisher exact test, FDR significance criterion, Benjamini-Hochberg correction, p val <0.01. P^{(a)} pval of Autoimmune patients *versus* Healthy donors. P^{(b)} val of Hepatitis C patients *versus* Healthy donors. n.s.: not significant. | | | | | | | |

Only 17 auto-antigens out of 24 elicited responses with significantly higher mean fluorescence intensity in autoimmune patients as compared to non autoimmune patients (pval <0.01, Fig. 6). Interestingly, approximately more than 60% of patients sera were simultaneously reactive against at least five of these autoantigens (Table 7).

**Table 7: Sera reacting against multiple autoimmune recognized antigens by microarray**

| | n. Obs (%) | | |
|---|---|---|---|
| **Positive Protons*** | **HD** | **HCV** | **Al** |
| *5* | 1/78 **(1)** | 33/122 **(27)** | 21/30 **(70)** |
| *at least 4* | 2/78 **(3)** | 34/122 **(28)** | 21/30 **(70)** |
| *at least 3* | 3/78 **(4)** | 40/122 **(33)** | 23/30 **(77)** |

| | | | |
|---|---|---|---|
| *Number of proteins simultaneously recognized by the same serum. **HD:** healthy donor; **AI:** autoimmune liver disease patients; **HCV:** patients with Hepatitis C Virus (w/wo autoimmunity extrahepatic disorder). | | | |

### Example 4- Validation of autoantigens profile.

After having identified a total of 17 auto-antigens specific for autoimmune patients by protein microarray, the authors confirmed the results obtained by using the DELFIA^{®} assay method (*Materials and Methods*). Because several top antigens immunogenic in autoimmune liver diseases have been previously described, in particular Asialoglycoprotein receptor (ASGR-1), cytochrome P450db1 (CYP2D6), the authors included them in the study as additional control proteins.

In order to validate the data, a second panel of 70 sera (see Table 2, *Materials and Methods*), was evaluated for the presence of autoantibodies for the 17 auto-antigens obtained in the previous phases.

In particular, the authors analyzed sera of 10 patients with HCV infection, of 10 patients with hepatitis C and extrahepatic autoimmune diseases, of 10 patients with autoimmine hepatitis (AIH) and of 10 patients with primary biliary cirrhosis (PBC). As negative controls, they also included 10 sera samples from patients with HBV and 20 samples from healthy donors. All sera were tested at a dilution of 1:300 as described in *Materials and Methods*, and the antigen-specific IgG responses to each of the proteins present on the plate were measured by the DELFIA^{®} time-resolved fluorescence. Reproducibility of the results was confirmed using duplicate sample of selected sera. Fluorescence intensity values higher than the mean plus two standard deviations for each serum from the healthy donor groups were considered to be positive. Using this method the authors found that sera of 13 out of the 20 patients with autoimmune liver disease (65%) were positive for at least five of the 17 autoantigens, but no serum IgG from healthy controls. 9 out of 20 and 11 out of 20 reacted with AGPR1 and CYP2D6 respectively (Table 8). These results were comparable to the screening of microarray (Table 7).

**Table 8: Sera reacting against multiple autoimmune recognized antigens by DELFIA^{®}**

| | n. Obs (%) | | | |
|---|---|---|---|---|
| **Positive Proteins'** | **HD** | **HBV** | **HCV** | **Al** |
| *5* | 0/20 **(0)** | 0/10 **(0)** | 2/20 **(10)** | 13/20 **(65)** |
| *at least 4* | 0/20 **(0)** | 0/10 **(10)** | 2/20 **(10)** | 13/20 **(65)** |
| *at least 3* | 1/20 **(5)** | 1/10 **(10)** | 2/20 **(10)** | 14/20 **(70)** |

| | | | | |
|---|---|---|---|---|
| *Number of proteins simultaneously recognized by the same serum. **HD:** healthy donor; **Al:** autoimmune liver disease patients; **HCV:** patients with Hepatitis C Virus (w/wo autoimmunity extrahepatic disorder).; **HBV:** Hepatitis B Virus patients | | | | |

The antigens showing the highest score in autoimmune liver disease sera were immunogenic in more than 45% of patients respectively, as compared to less than 16% of healthy donors. Most other autoantigens were recognized by less than 40% of autoimmune liver disease patients serum samples, or recognized also by sera from HCV patients serum sample, and therefore were not further selected (Table 9).

**Table 9: Candidate auto-antigens found immunogenic in autoimmune liver disease patients as compared to healthy donor sera and others groups**

| **Description** | **Protein** | **Numₛₑᵣₐ₊/num_{Tot}(%)** | | | |
|---|---|---|---|---|---|
| | **Id** | Al | HD | HCV | HBV |
| interleukin 4 receptor | YM0078 | 13/20 | 0/20 | 1/20 | 0/10 |
| | | (65) | (0) | (5) | (0) |
| lysozyme G-like 1 | YM0120 | 10/20 | 1/20 | 2/20 | 1/10 |
| | | (50) | (5) | (10) | (10) |
| chromosome 19 open reading frame 47 | YM0736 | 2/20 | 0/20 | 0/20 | 0/10 |
| | | (10) | (0) | (0) | (0) |
| AILT5830 | YM1451 | 11/20 | 3/20 | 5/20 | 3/10 |
| | | (55) | (15) | (25) | (30) |
| hypothetical protein DKFZp667F0711 | YM1503 | 6/20 | 0/20 | 1/20 | 0/10 |
| | | (30) | (0) | (5) | (0) |
| zinc finger protein 618 | YM1535 | 8/20 | 0/20 | 1/20 | 0/10 |
| | | (40) | (0) | (5) | (0) |
| hypothetical protein XP_934154 | YM1602 | 10/20 | 0/20 | 1/20 | 0/10 |
| | | (50) | (0) | (5) | (0) |
| chromosome 17 open reading frame 99 | YM1651 | 10/20 | 1/20 | 2/20 | 0/10 |
| | | (50) | (5) | (10) | (0) |
| chromosome 17 open reading frame 99 | YM1652 | 9/20 | 1/20 | 2/20 | 0/10 |
| | | (45) | (5) | (10) | (0) |
| meteorin, glial cell differentiation regulator-like | YM1672 | 6/20 | 0/20 | 1/20 | 0/10 |
| | | (30) | (0) | (5) | (0) |
| LP5624 | YM 1708 | 17/20 | 6/20 | 6/20 | 3/10 |
| | | (85) | (30) | (30) | (30) |
| carboxypeptidase X (M14 family), member 2 | YM1882 | 9/20 | 0/20 | 1/20 | 0/10 |
| | | (45) | (0) | (5) | (0) |
| vitamin D (1,25- dihydroxyvitamin D3) receptor | YM1980 | 0/20 | 0/20 | 0/20 | 0/10 |
| | | (0) | (0) | (0) | (0) |
| chondroadherin-like | YM 1989 | 5/20 | 0/20 | 0/20 | 0/10 |
| | | (25) | (0) | (0) | (0) |
| adenomatosis polyposis coli down-regulated 1-like | YM2046 | 13/20 | 0/20 | 1/20 | 0/10 |
| | | (65) | (0) | (5) | (0) |
| WAP four-disulfide core domain 10B | YM2671 | 10/20 | 0/20 | 2/20 | 0/10 |
| | | (50) | (0) | (10) | (0) |
| chondroadherin | YM2814 | 0/20 | 0/20 | 0/20 | 0/10 |
| | | (0) | (0) | (0) | (0) |

### Example 5- Confirmation of the specificity of the autoantigens profile for clinical applications.

The authors then asked if the detected autoantibodies were in common between Al and HCV patients, or specific to the Al group. Using as cut-off a value of two times the SD above the mean of healthy donor groups, they calculated the sensitivity and specificity values for each top antigen. From the screening measured by DELFIA^{®}, 4 new candidate autoantigens (YM0078, YM1602, YM2046, YM2671) were found highly specific towards the autoimmune samples showing a sensitivity value that ranged from 50% to 65% (Table 10). This sensitivity was significantly higher than the one measured for the remaining candidate autoantigens, which also showed a slight cross-reactivity, with sera from healthy donors and others controls as showed in Table 10.

**Table 10: Candidate auto-antigens found immunogenic in autoimmune-specific liver disease sera in DELFIA^{®}**

| **Prot. Id** | sensitivity^{a} | specificity^{b} | accuracy | *P values |
|---|---|---|---|---|
| | | | | |
| YM00078 | 65% | 100% | 83% | < 0.0001 |
| YM01602 | 50% | 100% | 75% | 0.0004 |
| YM02046 | 65% | 100% | 83% | < 0.0001 |
| YM02671 | 50% | 100% | 75% | 0.0004 |
| CYp450 | 55% | 90% | 73% | 0.0057 |
| AGPR-1 | 45% | 90% | 68% | 0.0310 |

| | | | | |
|---|---|---|---|---|
| ^{(a)}Sensitivity is defined as % of positive autoimmune patients. ^{(b)}Specificity is defined as % of negative healthy donors. *P values of autoimmune patients *versus* healthy calculated with two-sided Fisher's exact test. | | | | |

The authors next asked whether a combination panel of these four autoantigens may exhibit a higher sensitivity and specificity as compared to each single autoantigen.

The ability of the panel of 4 autoantigens to discriminate between autoimmune liver samples and healthy donor samples was highly significant (P<0.0001), showing a sensitivity equal to 0.8 (95 percent confidence interval, 0.56 to 0.94) and a specificity equal to 1 (95 percent confidence interval, 0.83 to 1.00) (Figure 7).

Finally, no differences were observed when we compared the two different cohort sera of HCV affected either with or without autoimmune diseases respectively. Moreover the sera from the negative controls as such as from HBV and from healthy donors did not result positive in this analysis (data not shown).

### REFERENCES

1. Bogdanos DP, Mieli-Vergani G, Vergani D. Autoantibodies and their antigens in autoimmune hepatitis. Semin Liver Dis. 2009 Aug;29(3):241-53.
2. Muratori L, Muratori P, Granito A, Pappas G, Cassani F, Lenzi M. Current topics in autoimmune hepatitis. Dig Liver Dis. 2010 Nov;42(11):757-64.
3. Muratori L, Granito A, Muratori P, Pappas G, Bianchi FB. Antimitochondrial antibodies and other antibodies in primary biliary cirrhosis: diagnostic and prognostic value. Clin Liver Dis. 2008 May;12(2):261-76; vii.
4. Song Q, Liu G, Hu S, Zhang Y, Tao Y, Han Y, Zeng H, Huang W, Li F, Chen P, Zhu J, Hu C, Zhang S, Li Y, Zhu H, Wu L. Novel autoimmune hepatitis-specific autoantigens identified using protein microarray technology. J Proteome Res. 2010 Jan;9(1):30-9.
5. Bombaci M, Grifantini R, Mora M, Reguzzi V, Petracca R, Meoni E, Balloni S, Zingaretti C, Falugi F, Manetti AG, Margarit I, Musser JM, Cardona F, Orefici G, Grandi G, Bensi G. Protein array profiling of tic patient sera reveals a broad range and enhanced immune response against Group A Streptococcus antigens. PLoS One. 2009 Jul 22;4(7):e6332.
6. Harris JW, Stocker H. Maximum. Likelihood Method. Handbook of Mathematics and Computational Science. 1998;1:824.
7. Frulloni L, Lunardi C, Simone R, Dolcino M, Scattolini C, Falconi M, Benini L, Vantini I, Corrocher R, Puccetti A. Identification of a novel antibody associated with autoimmune pancreatitis. N Engl J Med. 2009 Nov 26;361 (22):2135-42.

## Claims

1. An *in vitro* method of diagnosis or prognosis of a liver autoimmune disorder in a subject, comprising the steps of:
a) contacting a biological sample from the subject with at least one protein selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof, under conditions appropriate for binding of autoantibodies present in the biological sample to said protein or immunological fragments or functional equivalents thereof and
b) detecting the presence of bound antibodies, wherein said detection is indicative of a liver autoimmune disorder or of a risk of developing a liver autoimmune disorder.

2. The method according to claim 1 wherein step a) is performed by contacting said biological sample with at least three proteins selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof.

3. The method according to claim 2 wherein step a) is performed by contacting said biological sample with at least four proteins selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof.

4. The method according to claims 1 to 3 wherein said proteins or immunological fragments or functional equivalents thereof are comprised in the amino acid sequences of SEQ ID No. 1, 8, 17 and 22.

5. The method according to claim 3 or 4 wherein step a) is performed by contacting said biological sample with at least five proteins selected from the group of 17 proteins having the amino acid sequences SEQ ID No. 1, 2, 3, 5, 6, 7, 8, 9, 10, 11, 12, 14, 15, 16, 17, 22, 24 or immunological fragments or functional equivalents thereof.

6. The method according to claim 5 wherein step a) is performed by contacting said biological sample with at least a further protein of the group of proteins or immunological fragments or functional equivalents thereof comprised in the amino acid sequences SEQ ID No. 2, 3, 5, 6, 7, 9, 10, 11, 12, 14, 15, 16, 24.

7. The method according to any of previous claims wherein the biological sample is comprised in the group of blood, serum, plasma, urine, saliva, mucus, or fractions thereof.

8. The method according to any of previous claims wherein the liver autoimmune disorder is an Autoimmune Hepatitis (AIH) or a Primary Biliary Cirrhosis (PBC) disorder.

9. The method of any one of previous claims wherein the biological sample is from an adult or from an adolescent.

10. The method according to any of previous claims wherein the detection of said bound autoantibodies is performed by means of binding to specific ligands.

11. The method according to claim 10 wherein the ligands are conjugated with revealing means.

12. A method of monitoring an autoimmune liver disorder after treatment with surgery and/or therapy in a subject with said autoimmune liver disorder, comprising the step of following the modulation of proteins as claimed in any of previous claims.

13. The method of any one of previous claims, wherein said proteins or functional equivalents thereof are displayed on one or more protein microarrays.

14. A protein microarray comprising at least the proteins as defined in any of claims 1 to 6 or immunological fragments or functional equivalents thereof.

15. A solid support for an immunodiagnostic assay comprising at least the proteins as defined in any of claims 1 to 6 or immunological fragments or functional equivalents thereof.

16. An immunodiagnostic kit comprising the solid support as in claim 15 and detecting means.


SEQUENCE LISTING

<110>  Istituto Nazionale di Genetica Molecolare - INGM

<120>  BIOMARKERS FOR AUTOIMMUNE LIVER DISEASES

<130>  BE111868

<160>  24

<170>  PatentIn version 3.5

<210>  1
<211>  97
<212>  PRT
<213>  Homo sapiens

<400>  1

Pro Arg Ala Pro Gly Asn Leu Thr Val His Thr Asn Val Ser Asp Thr
1               5                   10                  15

Leu Leu Leu Thr Trp Ser Asn Pro Tyr Pro Pro Asp Asn Tyr Leu Tyr
            20                  25                  30

Asn His Leu Thr Tyr Ala Val Asn Ile Trp Ser Glu Asn Asp Pro Ala
        35                  40                  45

Asp Phe Arg Ile Tyr Asn Val Thr Tyr Leu Glu Pro Ser Leu Arg Ile
    50                  55                  60

Ala Ala Ser Thr Leu Lys Ser Gly Ile Ser Tyr Arg Ala Arg Val Arg
65                  70                  75                  80

Ala Trp Ala Gln Cys Tyr Asn Thr Thr Trp Ser Glu Trp Ser Pro Ser
                85                  90                  95

Thr

<210>  2
<211>  175
<212>  PRT
<213>  Homo sapiens

<400>  2

Ser Asn Trp Gly Cys Tyr Gly Asn Ile Gln Ser Leu Asp Thr Pro Gly
1               5                   10                  15

Ala Ser Cys Gly Ile Gly Arg Arg His Gly Leu Asn Tyr Cys Gly Val
            20                  25                  30

Arg Ala Ser Glu Arg Leu Ala Glu Ile Asp Met Pro Tyr Leu Leu Lys
        35                  40                  45

Tyr Gln Pro Met Met Gln Thr Ile Gly Gln Lys Tyr Cys Met Asp Pro
    50                  55                  60

Ala Val Ile Ala Gly Val Leu Ser Arg Lys Ser Pro Gly Asp Lys Ile
65                  70                  75                  80

Leu Val Asn Met Gly Asp Arg Thr Ser Met Val Gln Asp Pro Gly Ser
                85                  90                  95

Gln Ala Pro Thr Ser Trp Ile Ser Glu Ser Gln Val Ser Gln Thr Thr
            100                 105                 110

Glu Val Leu Thr Thr Arg Ile Lys Glu Ile Gln Arg Arg Phe Pro Thr
        115                 120                 125

Trp Thr Pro Asp Gln Tyr Leu Arg Gly Gly Leu Cys Ala Tyr Ser Gly
    130                 135                 140

Gly Ala Gly Tyr Val Arg Ser Ser Gln Asp Leu Ser Cys Asp Phe Cys
145                 150                 155                 160

Asn Asp Val Leu Ala Arg Ala Lys Tyr Leu Lys Arg His Gly Phe
                165                 170                 175

<210>  3
<211>  237
<212>  PRT
<213>  Homo sapiens

<400>  3

Met Phe Val Asp Asn Arg Ile Gln Lys Ser Met Leu Leu Asp Leu Asn
1               5                   10                  15

Lys Glu Ile Met Asn Glu Leu Gly Val Thr Val Val Gly Asp Ile Ile
            20                  25                  30

Ala Ile Leu Lys His Ala Lys Val Val His Arg Gln Asp Met Cys Lys
        35                  40                  45

Ala Ala Thr Glu Ser Val Pro Cys Ser Pro Ser Pro Leu Ala Gly Glu
    50                  55                  60

Ile Arg Arg Gly Thr Ser Ala Ala Ser Arg Met Ile Thr Asn Ser Leu

65                  70                  75                  80

Asn His Asp Ser Pro Pro Ser Thr Pro Pro Arg Arg Pro Asp Thr Ser
                85                  90                  95

Thr Ser Lys Ile Ser Val Thr Val Ser Asn Lys Met Ala Ala Lys Ser
            100                 105                 110

Ala Lys Ala Thr Ala Ala Leu Ala Arg Arg Glu Glu Glu Ser Leu Ala
        115                 120                 125

Val Pro Ala Lys Arg Arg Arg Val Thr Ala Glu Met Glu Gly Lys Tyr
    130                 135                 140

Val Ile Asn Met Pro Lys Gly Thr Thr Pro Arg Thr Arg Lys Ile Leu
145                 150                 155                 160

Glu Gln Gln Gln Ala Ala Lys Gly Leu His Arg Thr Ser Val Phe Asp
                165                 170                 175

Arg Leu Gly Ala Glu Thr Lys Ala Asp Thr Thr Thr Gly Ser Lys Pro
            180                 185                 190

Thr Gly Val Phe Ser Arg Leu Gly Ala Thr Pro Glu Thr Asp Glu Asp
        195                 200                 205

Leu Ala Trp Asp Ser Asp Asn Asp Ser Ser Ser Ser Val Leu Gln Tyr
    210                 215                 220

Ala Gly Val Leu Lys Lys Leu Gly Arg Gly Pro Ala Lys
225                 230                 235

<210>  4
<211>  79
<212>  PRT
<213>  Homo sapiens

<400>  4

Ser Gln Gly Val Cys Ser Lys Gln Thr Leu Val Val Pro Leu His Tyr
1               5                   10                  15

Asn Glu Ser Tyr Ser Gln Pro Val Tyr Lys Pro Tyr Leu Thr Leu Cys
            20                  25                  30

Ala Gly Arg Arg Ile Cys Ser Thr Tyr Arg Thr Met Tyr Arg Val Met
        35                  40                  45

Trp Arg Glu Val Arg Arg Glu Val Gln Gln Thr His Ala Val Cys Cys
    50                  55                  60

Gln Gly Trp Lys Lys Arg His Pro Gly Ala Leu Thr Cys Glu Ala
65                  70                  75

<210>  5
<211>  76
<212>  PRT
<213>  Homo sapiens

<400>  5

His Glu Ala His Lys Thr Ser Leu Ser Ser Trp Lys His Asp Gln Asp
1               5                   10                  15

Trp Ala Asn Val Ser Asn Met Thr Phe Ser Asn Gly Lys Leu Arg Val
            20                  25                  30

Lys Gly Ile Tyr Tyr Arg Asn Ala Asp Ile Cys Ser Arg His Arg Val
        35                  40                  45

Thr Ser Ala Gly Leu Thr Leu Gln Asp Leu Gln Leu Trp Cys Asn Leu
    50                  55                  60

Arg Ser Val Ala Arg Gly Gln Ile Pro Ser Thr Leu
65                  70                  75

<210>  6
<211>  101
<212>  PRT
<213>  Homo sapiens

<400>  6

Gly Val Ala Glu Phe His Met Ser Leu Thr Val Ser Cys Pro Asp Pro
1               5                   10                  15

Thr Pro Ser Thr Asp Pro Gln Gly Arg His Asn Arg Glu Pro Ile Leu
            20                  25                  30

Gly Arg Asp Asp Asp Phe Met Cys Lys Gln Val Lys Phe Arg Met Cys
        35                  40                  45

Val Val Gly Arg Asp Gly Asn Ala Gln Ser Ser Val Arg Tyr Thr Gly
    50                  55                  60

Pro Leu Tyr Arg Arg Lys Ile Arg Thr Glu Phe Leu Phe Val Val Phe

65                  70                  75                  80

Leu Leu Glu Thr Arg Glu Leu Lys Pro Gln Val Asn Lys Asn Val Gln
                85                  90                  95

Gly Thr Arg Pro Ser
            100

<210>  7
<211>  92
<212>  PRT
<213>  Homo sapiens

<400>  7

<210>  8
<211>  92
<212>  PRT
<213>  Homo sapiens

<400>  8

Ala Thr Gly Ile Leu Ile Cys Met Thr Lys Asn Leu Glu Ser Val His
1               5                   10                  15

Ser Ile Val Leu Ala His Ser Cys Tyr His His Glu Asn Lys Pro Arg
            20                  25                  30

Pro Asp Cys Cys Phe Gln Gln Lys Ile Arg Asp Thr Lys Ser Lys Val
        35                  40                  45

Glu Leu Pro Arg His Ala His Ala Arg Leu Thr Asn Pro Gln Leu Thr

50                  55                  60

His Arg Ser Met Lys Ile Asn Asp Cys Cys Ile Lys Pro Leu Arg Phe
65                  70                  75                  80

Gly Val Thr Cys Tyr Ala Ala Phe Cys Asp Asn Asn
                85                  90

<210>  9
<211>  237
<212>  PRT
<213>  Homo sapiens

<400>  9

Ala Arg Glu Glu Glu Ile Thr Pro Val Val Ser Ile Ala Tyr Lys Val
1               5                   10                  15

Leu Glu Val Phe Pro Lys Gly Arg Trp Val Leu Ile Thr Cys Cys Ala
            20                  25                  30

Pro Gln Pro Pro Pro Pro Ile Thr Tyr Ser Leu Cys Gly Thr Lys Asn
        35                  40                  45

Ile Lys Val Ala Lys Lys Val Val Lys Thr His Glu Pro Ala Ser Phe
    50                  55                  60

Asn Leu Asn Val Thr Leu Lys Ser Ser Pro Asp Leu Leu Thr Tyr Phe
65                  70                  75                  80

Cys Trp Ala Ser Ser Thr Ser Gly Ala His Val Asp Ser Ala Arg Leu
                85                  90                  95

Gln Met His Trp Glu Leu Trp Ser Lys Pro Val Ser Glu Leu Arg Ala
            100                 105                 110

Asn Phe Thr Leu Gln Asp Arg Gly Ala Gly Pro Arg Val Glu Met Ile
        115                 120                 125

Cys Gln Ala Ser Ser Gly Ser Pro Pro Ile Thr Asn Ser Leu Ile Gly
    130                 135                 140

Lys Asp Gly Gln Val His Leu Gln Gln Arg Pro Cys His Arg Gln Pro
145                 150                 155                 160

Ala Asn Phe Ser Phe Leu Pro Ser Gln Thr Ser Asp Trp Phe Trp Cys
                165                 170                 175

Gln Ala Ala Asn Asn Ala Asn Val Gln His Ser Ala Leu Thr Val Val
            180                 185                 190

Pro Pro Gly Gly Asp Gln Lys Met Glu Asp Trp Gln Gly Pro Leu Glu
        195                 200                 205

Ser Pro Ile Leu Ala Leu Pro Leu Tyr Arg Ser Thr Arg Arg Leu Ser
    210                 215                 220

Glu Glu Glu Phe Gly Gly Phe Arg Ile Gly Asn Gly Glu
225                 230                 235

<210>  10
<211>  124
<212>  PRT
<213>  Homo sapiens

<400>  10

Val Ala Lys Lys Val Val Lys Thr His Glu Pro Ala Ser Phe Asn Leu
1               5                   10                  15

Asn Val Thr Leu Lys Ser Ser Pro Asp Leu Leu Thr Tyr Phe Cys Trp
            20                  25                  30

Ala Ser Ser Thr Ser Gly Ala His Val Asp Ser Ala Arg Leu Gln Met
        35                  40                  45

His Trp Glu Leu Trp Ser Lys Pro Val Ser Glu Leu Arg Ala Asn Phe
    50                  55                  60

Thr Leu Gln Asp Arg Gly Ala Gly Pro Arg Val Glu Met Ile Cys Gln
65                  70                  75                  80

Ala Ser Ser Gly Ser Pro Pro Ile Thr Asn Ser Leu Ile Gly Lys Asp
                85                  90                  95

Gly Gln Val His Leu Gln Gln Arg Pro Cys His Arg Gln Pro Ala Asn
            100                 105                 110

Phe Ser Phe Leu Pro Ser Gln Thr Ser Asp Trp Phe
        115                 120

<210>  11
<211>  237
<212>  PRT

<213>  Homo sapiens

<400>  11

Ala Gln Tyr Ser Ser Asp Arg Cys Ser Trp Lys Gly Ser Gly Leu Thr
1               5                   10                  15

His Glu Ala His Arg Lys Glu Val Glu Gln Val Tyr Leu Arg Cys Ala
            20                  25                  30

Ala Gly Ala Val Glu Trp Met Tyr Pro Thr Gly Ala Leu Ile Val Asn
        35                  40                  45

Leu Arg Pro Asn Thr Phe Ser Pro Ala Arg His Leu Thr Val Cys Ile
    50                  55                  60

Arg Ser Phe Thr Asp Ser Ser Gly Ala Asn Ile Tyr Leu Glu Lys Thr
65                  70                  75                  80

Gly Glu Leu Arg Leu Leu Val Pro Asp Gly Asp Gly Arg Pro Gly Arg
                85                  90                  95

Val Gln Cys Phe Gly Leu Glu Gln Gly Gly Leu Phe Val Glu Ala Thr
            100                 105                 110

Pro Gln Gln Asp Ile Gly Arg Arg Thr Thr Gly Phe Gln Tyr Glu Leu
        115                 120                 125

Val Arg Arg His Arg Ala Ser Asp Leu His Glu Leu Ser Ala Pro Cys
    130                 135                 140

Arg Pro Cys Ser Asp Thr Glu Val Leu Leu Ala Val Cys Thr Ser Asp
145                 150                 155                 160

Phe Ala Val Arg Gly Ser Ile Gln Gln Val Thr His Glu Pro Glu Arg
                165                 170                 175

Gln Asp Ser Ala Ile His Leu Arg Val Ser Arg Leu Tyr Arg Gln Lys
            180                 185                 190

Ser Arg Val Phe Glu Pro Val Pro Glu Gly Asp Gly His Trp Gln Gly
        195                 200                 205

Arg Val Arg Thr Leu Leu Glu Cys Gly Val Arg Pro Gly His Gly Asp
    210                 215                 220

Phe Leu Phe Thr Gly His Met His Phe Gly Glu Ala Arg

225                 230                 235

<210>  12
<211>  95
<212>  PRT
<213>  Homo sapiens

<400>  12
   Met Phe Gly Val Leu Glu Gly Ala Gln Ala Asn Ser Glu Asn Trp Ile
   1               5                   10                  15
   Ala Pro Ser Gly Pro Trp Ala Leu Gly Leu Trp Ser Ser Leu Tyr Phe
               20                  25                  30
   Leu Leu Phe Ser Thr Leu Glu Gly Arg Gly Gly Arg Val Leu Ser Gln
           35                  40                  45
   Ser Cys Ser Met Ala Val Ala Ala Ala Ser Trp Ile Ser Arg Glu Asn
       50                  55                  60
   Ala Arg Ser Val Lys Arg Ser Tyr Met Gln Ser Ser Pro Gln Arg Pro
   65                  70                  75                  80
   Lys Glu Pro Arg Asn Gln Arg Thr Ser His Thr Thr Pro Val Cys
                   85                  90                  95

<210>  13
<211>  205
<212>  PRT
<213>  Homo sapiens

<400>  13

Ser Gly Phe Thr Ala Leu His Trp Ala Ala Lys Ser Gly Asp Gly Glu
1               5                   10                  15

Met Ala Leu Gln Leu Val Glu Val Ala Arg Arg Ser Gly Ala Pro Val
            20                  25                  30

Asp Val Asn Ala Arg Ser His Gly Gly Tyr Thr Pro Leu His Leu Ala
        35                  40                  45

Ala Leu His Gly His Glu Asp Ala Ala Val Leu Leu Val Val Arg Leu
    50                  55                  60

Gly Ala Gln Val His Val Arg Asp His Ser Gly Arg Arg Ala Tyr Gln
65                  70                  75                  80

Tyr Leu Arg Pro Gly Ser Ser Tyr Ala Leu Arg Arg Leu Leu Gly Asp

                85                  90                  95

Pro Gly Leu Arg Gly Thr Thr Glu Pro Asp Ala Thr Gly Gly Gly Ser
            100                 105                 110

Gly Ser Leu Ala Ala Arg Arg Pro Val Gln Val Ala Ala Thr Ile Leu
        115                 120                 125

Ser Ser Thr Thr Ser Ala Phe Leu Gly Val Leu Ala Asp Asp Leu Met
    130                 135                 140

Leu Gln Asp Leu Ala Arg Gly Leu Lys Lys Ser Ser Ser Phe Ser Lys
145                 150                 155                 160

Phe Leu Ser Ala Ser Pro Met Ala Pro Arg Lys Lys Thr Lys Ile Arg
                165                 170                 175

Gly Gly Leu Pro Ala Phe Ser Glu Ile Ser Arg Arg Pro Thr Pro Gly
            180                 185                 190

Pro Leu Ala Gly Leu Val Pro Ser Phe Pro Pro Thr Thr
        195                 200                 205

<210> 14
<211> 125
<212> PRT
<213> Homo sapiens

<400> 14

Ser Leu Ile Val Phe Met Glu Gln Val His Arg Gly Ile Lys Gly Leu
1               5                   10                  15

Val Arg Asp Ser His Gly Lys Gly Ile Pro Asn Ala Ile Ile Ser Val
            20                  25                  30

Glu Gly Ile Asn His Asp Ile Arg Thr Ala Asn Asp Gly Asp Tyr Trp
        35                  40                  45

Arg Leu Leu Asn Pro Gly Glu Tyr Val Val Thr Ala Lys Ala Glu Gly
    50                  55                  60

Phe Thr Ala Ser Thr Lys Asn Cys Met Val Gly Tyr Asp Met Gly Ala
65                  70                  75                  80

Thr Arg Cys Asp Phe Thr Leu Ser Lys Thr Asn Met Ala Arg Ile Arg
                85                  90                  95

Glu Ile Met Glu Lys Phe Gly Lys Gln Pro Val Ser Leu Pro Ala Arg
            100                 105                 110

Arg Leu Lys Leu Arg Gly Gln Lys Arg Arg Gln Arg Gly
        115                 120                 125

<210>  15
<211>  99
<212>  PRT
<213>  Homo sapiens

<400>  15

Gly Ser Leu Ser Pro Thr Lys Tyr Asn Leu Leu Glu Leu Lys Glu Ser
1               5                   10                  15

Cys Ile Arg Asn Gln Asp Cys Glu Thr Gly Cys Cys Gln Arg Ala Pro
            20                  25                  30

Asp Asn Cys Glu Ser His Cys Ala Glu Lys Gly Ser Glu Gly Ser Leu
        35                  40                  45

Cys Gln Thr Gln Val Phe Phe Gly Gln Tyr Arg Ala Cys Pro Cys Leu
    50                  55                  60

Arg Asn Leu Thr Cys Ile Tyr Ser Lys Asn Glu Lys Trp Leu Ser Ile
65                  70                  75                  80

Ala Tyr Gly Arg Cys Gln Lys Ile Gly Arg Gln Lys Leu Ala Lys Lys
                85                  90                  95

Met Phe Phe

<210>  16
<211>  98
<212>  PRT
<213>  Homo sapiens

<400>  16

Ser Gln Leu Glu Leu Ile Asp Leu Ser Ser Asn Pro Phe His Cys Asp
1               5                   10                  15

Cys Gln Leu Leu Pro Leu His Arg Trp Leu Thr Gly Leu Asn Leu Arg
            20                  25                  30

Val Gly Ala Thr Cys Ala Thr Pro Pro Asn Ala Arg Gly Gln Arg Val

        35                  40                  45

Lys Ala Ala Ala Ala Val Phe Glu Asp Cys Pro Gly Trp Ala Ala Arg
    50                  55                  60

Lys Ala Lys Arg Thr Pro Ala Ser Arg Pro Ser Ala Arg Arg Thr Pro
65                  70                  75                  80

Ile Lys Gly Arg Gln Cys Gly Ala Asp Lys Val Gly His Gly Ala Gly
                85                  90                  95

Gly Val

<210> 17
<211> 112
<212> PRT
<213> Homo sapiens

<400> 17

Gly Glu Ala Gly Gly Ser Cys Leu Arg Trp Glu Pro His Cys Gln Gln
1               5                   10                  15

Pro Leu Pro Asp Arg Val Pro Ser Thr Ala Ile Leu Pro Pro Arg Leu
            20                  25                  30

Asn Gly Pro Trp Ile Ser Thr Gly Cys Glu Val Arg Pro Gly Pro Glu
        35                  40                  45

Phe Leu Thr Arg Ala Tyr Thr Phe Tyr Pro Ser Arg Leu Phe Arg Ala
    50                  55                  60

His Gln Phe Tyr Tyr Glu Asp Pro Phe Cys Gly Glu Pro Ala His Ser
65                  70                  75                  80

Leu Leu Val Lys Gly Lys Val Arg Leu Arg Arg Ala Ser Trp Val Thr
                85                  90                  95

Arg Gly Ala Thr Glu Ala Asp Tyr His Leu His Lys Val Gly Ile Val
            100                 105                 110

<210> 18
<211> 64
<212> PRT
<213> Homo sapiens

<400> 18

Ala Thr Lys Leu Val Thr Cys Pro Ala Pro Arg Gln Phe Ala Val Gly
1               5                   10                  15

Ala Phe Thr Ala Ala Gly Arg Ala Trp Leu Phe Ala Pro Ser Leu Gly
            20                  25                  30

Ala Ser Phe Ser Lys Leu Arg Ser Gln Gln Arg Ser Arg Asp Phe Arg
        35                  40                  45

Gly Arg Leu Phe Leu Arg Ala Glu Arg Arg Ala Gly Gly Phe Thr Ser
    50                  55                  60

<210>  19
<211>  237
<212>  PRT
<213>  Homo sapiens

<400>  19

Ser Ser Asn Leu Glu Gly Arg Thr Lys Ser Val Ile Arg Gln Thr Gly
1               5                   10                  15

Ser Ser Ala Glu Ile Thr Cys Asp Leu Ala Glu Gly Ser Thr Gly Tyr
            20                  25                  30

Ile His Trp Tyr Leu His Gln Glu Gly Lys Ala Pro Gln Arg Leu Leu
        35                  40                  45

Tyr Tyr Asp Ser Tyr Thr Ser Ser Val Val Leu Glu Ser Gly Ile Ser
    50                  55                  60

Pro Gly Lys Tyr Asp Thr Tyr Gly Ser Thr Arg Lys Asn Leu Arg Met
65                  70                  75                  80

Ile Leu Arg Asn Leu Ile Glu Asn Asp Ser Gly Val Tyr Tyr Cys Ala
                85                  90                  95

Thr Trp Asp Gly His Ser Asp Ser Asp Pro Pro Tyr Thr Thr Leu Lys
            100                 105                 110

Thr Cys Leu Val Ala Ala Ser Pro Arg Glu Glu Gly Met Arg Trp Ala
        115                 120                 125

Leu Leu Val Leu Leu Ala Phe Leu Ser Pro Ile Pro Ala Pro Pro Thr
    130                 135                 140

Val Phe Cys Ala Arg Asp His Phe Leu Val Glu Trp Asp Leu Ser Phe

145                 150                 155                 160

Glu Arg Ile Phe Tyr Ser Phe Ser Ser Gly Pro Phe Pro Ser Lys Ala
                165                 170                 175

Pro Glu Arg Lys Ala Cys Ser Gln Lys Ser Ser Asn Leu Glu Gly Arg
            180                 185                 190

Met Lys Ser Val Thr Arg Pro Thr Gly Ser Ser Ala Glu Ile Thr Cys
        195                 200                 205

Asp Leu Thr Val Ile Asn Ala Val Tyr Ile His Trp Tyr Leu Gln Gln
    210                 215                 220

Glu Gly Lys Thr Pro Gln His Leu Leu His Tyr Asp Val
225                 230                 235

<210>  20
<211>  145
<212>  PRT
<213>  Homo sapiens

<400>  20

Ala Gly Val Thr Gln Thr Pro Lys Phe His Val Leu Lys Thr Gly Gln
1               5                   10                  15

Ser Met Thr Leu Leu Cys Ala Gln Asp Met Asn His Glu Tyr Met Tyr
            20                  25                  30

Arg Tyr Arg Gln Asp Pro Gly Lys Gly Leu Arg Leu Ile Tyr Tyr Ser
        35                  40                  45

Val Ala Ala Ala Leu Thr Asp Lys Gly Glu Val Pro Asn Gly Tyr Asn
    50                  55                  60

Val Ser Arg Ser Asn Thr Glu Asp Phe Pro Leu Lys Leu Glu Ser Ala
65                  70                  75                  80

Ala Pro Ser Gln Thr Ser Val Tyr Phe Cys Ala Ser Ser Tyr Ser Thr
                85                  90                  95

Ala Leu Gln Gly Cys Leu Leu Ser Ala His Lys Gly Lys Gly Arg Cys
            100                 105                 110

Cys Pro Pro Pro Pro Pro Lys Thr Gln Gly Cys Pro Val Gln Arg Ser
        115                 120                 125

Leu His Gln Glu Pro Trp Asn Pro Glu Trp Pro Gln Val Ala Arg Thr
    130                 135                 140

Val
145

<210>  21
<211>  143
<212>  PRT
<213>  Homo sapiens

<400>  21

Ala Lys Val Thr Gln Thr Pro Gly His Leu Val Lys Gly Lys Gly Gln
1               5                   10                  15

Lys Thr Lys Met Asp Cys Thr Pro Glu Lys Gly His Thr Phe Val Tyr
            20                  25                  30

Trp Tyr Gln Gln Asn Gln Asn Lys Glu Phe Met Leu Leu Ile Ser Phe
        35                  40                  45

Gln Asn Glu Gln Val Leu Gln Glu Thr Glu Met His Lys Lys Arg Phe
    50                  55                  60

Ser Ser Gln Cys Pro Lys Asn Ala Pro Cys Ser Leu Ala Ile Leu Ser
65                  70                  75                  80

Ser Glu Pro Gly Asp Thr Ala Leu Tyr Leu Cys Ala Ser Ser Gln Ser
                85                  90                  95

Thr Ala Leu Lys Cys Gln Phe Leu Leu Ala His Lys Leu Val Thr Asp
            100                 105                 110

Pro Ala Gln Glu Ala Gly Asp Val Leu Gly Trp Lys Gly Val Thr Glu
        115                 120                 125

Asn Asn Trp Ser Gln Leu Lys Pro Gln Cys Asn Leu Thr Gln Gly
    130                 135                 140

<210>  22
<211>  50
<212>  PRT
<213>  Homo sapiens

<400>  22

Arg Asp Lys Met Arg Met Gln Arg Ile Lys Val Cys Glu Lys Arg Pro

1               5                   10                  15

Ser Ile Asp Leu Cys Ile His His Cys Ser Tyr Phe Gln Lys Cys Glu
            20                  25                  30

Thr Asn Lys Ile Cys Cys Ser Ala Phe Cys Gly Asn Ile Cys Met Ser
        35                  40                  45

Ile Leu
    50

<210>  23
<211>  237
<212>  PRT
<213>  Homo sapiens

<400>  23

Gln Leu Leu Met Tyr Gln Gln His Thr Ser His Tyr Asp Leu Glu Arg
1               5                   10                  15

Lys Gly Gly Tyr Leu Met Leu Ser Phe Ile Asp Phe Cys Pro Phe Ser
            20                  25                  30

Val Met Arg Leu Arg Ser Leu Pro Ser Pro Gln Arg Tyr Thr Arg Gln
        35                  40                  45

Glu Arg Tyr Arg Ala Arg Pro Pro Arg Val Leu Glu Arg Ser Gly Phe
    50                  55                  60

His Asn Glu Asn Ser Leu Ala Ile Tyr Gln Gly Leu Val Tyr Tyr Leu
65                  70                  75                  80

Leu Trp Leu His Ser Val Tyr Asp Lys Pro Tyr Ala Asp Pro Val His
                85                  90                  95

Asp Pro Thr Trp Arg Trp Trp Ala Asn Asn Lys Gln Asp Gln Asp Tyr
            100                 105                 110

Tyr Phe Phe Leu Ala Ser Asn Trp Arg Ser Ala Gly Gly Val Ser Ile
        115                 120                 125

Glu Met Asp Ser Tyr Glu Lys Ile Tyr Asn Leu Glu Ser Ala Tyr Glu
    130                 135                 140

Leu Pro Glu Arg Ile Phe Leu Asp Lys Gly Thr Glu Tyr Ser Phe Ala
145                 150                 155                 160

Ile Phe Leu Ser Ala Gln Gly His Ser Phe Arg Thr Gln Ser Glu Leu
                165                 170                 175

Gly Thr Ala Phe Gln Leu His Ser Gln Val Asp Val Gly Val Val Leu
            180                 185                 190

Ala Asp Pro Gly Cys Ile Glu Ala Ser Val Lys Gln Glu Val Leu Ile
        195                 200                 205

Asn Arg Asn Ser Val Leu Phe Ser Ile Thr Leu Lys Asp Lys Lys Leu
    210                 215                 220

Cys Tyr Asp Gln Gly Ile Ser Gly His His Leu Met Glu
225                 230                 235

<210>  24
<211>  140
<212>  PRT
<213>  Homo sapiens

<400>  24

Val Val Glu Glu Leu Lys Leu Ser His Asn Pro Leu Lys Ser Ile Pro
1               5                   10                  15

Asp Asn Ala Phe Gln Ser Phe Gly Arg Tyr Leu Glu Thr Leu Trp Leu
            20                  25                  30

Asp Asn Thr Asn Leu Glu Lys Phe Ser Asp Gly Ala Phe Leu Gly Val
        35                  40                  45

Thr Thr Leu Lys His Val His Leu Glu Asn Asn Arg Leu Asn Gln Leu
    50                  55                  60

Pro Ser Asn Phe Pro Phe Asp Ser Leu Glu Thr Leu Ala Leu Thr Asn
65                  70                  75                  80

Asn Pro Trp Lys Cys Thr Cys Gln Leu Arg Gly Leu Arg Arg Trp Leu
                85                  90                  95

Glu Ala Lys Ala Ser Arg Pro Asp Ala Thr Cys Ala Ser Pro Ala Lys
            100                 105                 110

Phe Lys Gly Gln His Ile Arg Asp Thr Asp Ala Phe Arg Ser Cys Lys
        115                 120                 125

Phe Pro Thr Lys Arg Ser Lys Lys Ala Gly Arg His

130                                    135                                    140